(19) Europäisches Patentamt
European Patent Office
Office européen des brevets

(11) Numéro de publication: **0 303 546 B1**

(12) **FASCICULE DE BREVET EUROPEEN**

(45) Date de publication de fascicule du brevet: **28.12.94**

(51) Int. Cl.5: **C07C 217/54**

(21) Numéro de dépôt: **88402095.9**

(22) Date de dépôt: **11.08.88**

Le dossier contient des informations techniques présentées postérieurement au dépôt de la demande et ne figurant pas dans le présent fascicule.

(54) **Procédé pour la O-alkylation de N-(hydroxy)aralkylphényléthanolamines.**

(30) Priorité: **12.08.87 FR 8711498**
**14.06.88 FR 8807948**

(43) Date de publication de la demande:
**15.02.89 Bulletin 89/07**

(45) Mention de la délivrance du brevet:
**28.12.94 Bulletin 94/52**

(84) Etats contractants désignés:
**AT BE CH DE ES FR GB GR IT LI LU NL SE**

(56) Documents cités:
**EP-A- 0 023 385**
**EP-A- 0 052 963**
**EP-A- 0 211 721**
**WO-A-81/00849**

(73) Titulaire: **SANOFI**
**32-34, rue Marbeuf**
**F-75008 Paris (FR)**
(84) Etats contractants désignés:
**BE CH DE ES FR GB GR LI LU NL SE AT**

(73) Titulaire: **MIDY S.p.A.**
**Via Piranesi 38**
**I-20137 Milano (IT)**
(84) Etats contractants désignés:
**IT**

(72) Inventeur: **Boigegrain, Robert**
**Lou Pradas N20 Jacou**
**F-34170 Castelnau le Lez (FR)**
Inventeur: **Cecchi, Roberto**
**Via Dei Tigli 1**
**I-20075 Lodi-Milan (IT)**
Inventeur: **Boveri, Sergio**
**Corso Repubblica, 48**
**I-15057 Tortona (Alessandria) (IT)**

(74) Mandataire: **Gillard, Marie-Louise et al**
**Cabinet Beau de Loménie**
**158, rue de l'Université**
**F-75340 Paris Cédex 07 (FR)**

**EP 0 303 546 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention concerne un procédé pour la O-alkylation de N-[(hydroxy)aralkyl]-phényléthanolamines, plus particulièrement pour l'alkylation de l'hydroxyle phénolique desdites phényléthanolamines (hydroxy)aralkylées, et des intermédiaires utilisés dans ce procédé.

Le brevet européen 211721 décrit des phényléthanolaminotétralines de formule

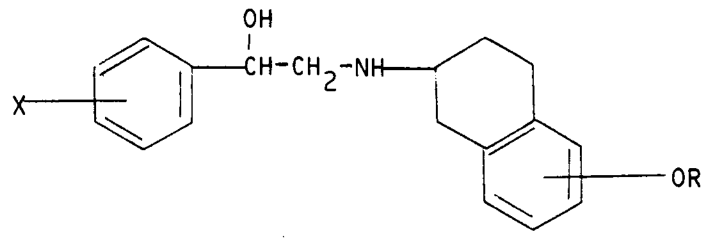

(A)

dans laquelle X représente l'hydrogène, un halogène, un groupe trifluorométhyle ou un groupe alkyle inférieur et R représente l'hydrogène; un groupe alkyle inférieur non substitué ou substitué par un groupe cycloalkyle contenant 3 à 7 atomes de carbone, un groupe hydroxy, alcoxy inférieur, carboxy ou carbalcoxy inférieur; un groupe cycloalkyle contenant 3 à 7 atomes de carbone ou un alcanoyle inférieur et leurs sels pharmaceutiquement acceptables.

Parmi les méthodes de préparation des produits de formule (A) ci-dessus est décrite une O-alkylation qui, à partir du 2-[(7-hydroxy-1,2,3,4-tétrahydronapht-2-yl)amino]-1-phényléthanol ou du 2-[(7-hydroxy-1,2,3,4-tétrahydronapht-2-yl)amino]-1-(3-chlorophényl)-éthanol,par réaction avec du bromoacétate d'éthyle donne le 2-[(7-carbéthoxyméthoxy-1,2,3,4-tétrahydronapht-2-yl)amino]-1-phényléthanol ou le 2-[(7-carbéthoxyméthoxy-1,2,3,4-tétrahydronapht-2-yl)amino]-1-(3-chlorophényl)éthanol. Cette réaction ne donne cependant pas de bons résultats, car le rendement en produit final est très faible.

On a maintenant trouvé que les produits ci-dessus peuvent être obtenus avec des rendements bien supérieurs en protégeant le groupe amino du composé de formule A ci-dessus, où R est l'hydrogène, avant la réaction d'alkylation et en débloquant ensuite le groupe amino.

Ainsi, la présente invention a pour objet un procédé pour la préparation de composés de formule

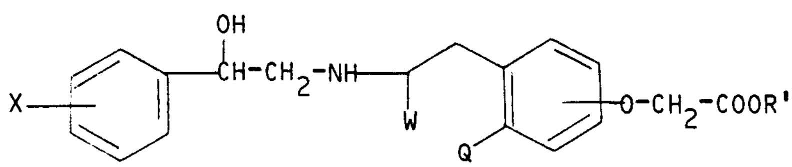

I

dans laquelle X représente l'hydrogène, un halogène, un groupe trifluorométhyle ou un groupe alkyle inférieur; W représente méthyle, Q représente hydrogène, ou W et Q, ensemble, forment un groupe éthylène et R′ représente un groupe alkyle inférieur; et de leurs sels pharmaceutiquement acceptables, caractérisé en ce que

(a) on protège le groupe amino d'un composé de formule

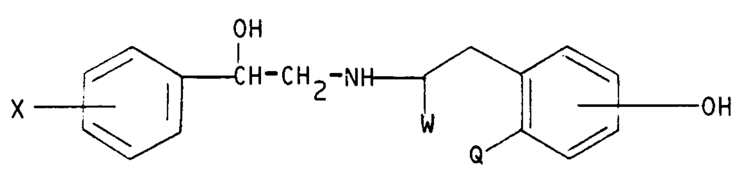

II

dans laquelle X, W et Q sont tels que définis ci-dessus,

(b) on soumet le composé ainsi obtenu de formule

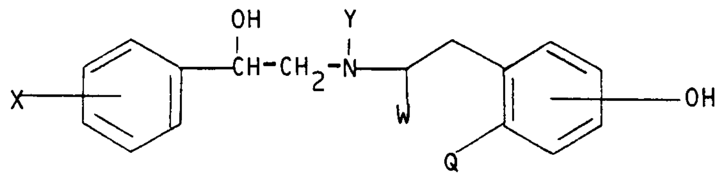

III

dans laquelle X, W et Q sont tels que définis ci-dessus et Y représente un groupe N-protecteur, à une alkylation avec un composé de formule Hal-$CH_2$-COOR′, dans laquelle R′ est tel que défini ci-dessus et Hal représente le chlore, le brome ou l'iode;

(c) on débloque le groupe amino protégé du composé ainsi obtenu de formule

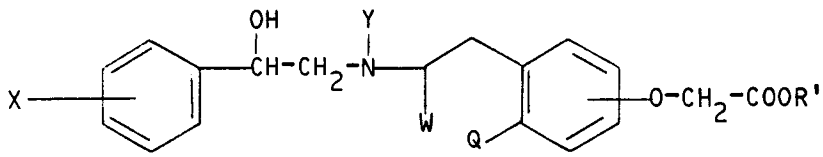

IV

dans laquelle X, Y, W, Q et R′ sont tels que définis ci-dessus, et on transforme éventuellement le produit ainsi obtenu en l'un de ses sels pharmaceutiquement acceptables.

Les groupes alkyles inférieurs contiennent de 1 à 4 atomes de carbone.

Parmi les halogènes, le fluor, le brome et surtout le chlore sont préférés.

Parmi les groupements protecteurs indiqués par Y, le groupe Boc (t.butyloxycarbonyle) et le groupe Z (benzyloxycarbonyle) sont avantageux.

Dans la présente description, les termes "tétraline" et "tétralone" se rapportent au 1,2,3,4-tétrahydronaphtalène.

Des composés préférés qui peuvent être obtenus selon le procédé de la présente invention sont ceux de formule I dans laquelle X est tel que défini ci-dessus, notamment 3-chloro, R′ représente un groupe méthyle ou éthyle et le groupe O-$CH_2$-COOR′ est, par rapport au substituant Q, en position méta lorsque Q est l'hydrogène et en position para lorsque W et Q, ensemble, forment un groupe éthylène.

Particulièrement préférés sont :

- la N-[(2R)-7-éthoxycarbonylméthoxy-1,2,3,4-tétrahydronapht-2-yl]-(2R)-2-(3-chlorophényl)-2-hydroxyéthanamine,
- la N-[(2S)-7-éthoxycarbonylméthoxy-1,2,3,4-tétrahydronapht-2-yl]-(2R)-2-(3-chlorophényl)-2-hydroxyéthanamine,
- la N-[(2R)-7-méthoxycarbonylméthoxy-1,2,3,4-tétrahydronapht-2-yl]-(2R)-2-(3-chlorophényl)-2-hydroxyéthanamine,

et leurs sels pharmaceutiquement acceptables.

Ces produits sont nouveaux et présentent des propriétés pharmacologiques particulièrement intéressantes.

Les composés de départ de formule II sont décrits en littérature, notamment dans les brevets EP 211721 (W + Q = éthylène), EP 66351 (W = méthyle, Q = hydrogène, X = trifluorométhyle), EP 70134 (W = méthyle, Q = hydrogène, X = halogène) ou ils peuvent aisément être préparés par des méthodes connues, notamment par celles des trois brevets mentionnés ci-dessus.

Le procédé de la présente invention est mis en oeuvre en faisant réagir un composé de formule II avec le réactif approprié pour la protection des groupes amino, comme décrit, par exemple, par M. Bodanszky et al., Peptide Synthesis, 2nd Edition, John Wiley & Sons, 1976, pages 18 à 49.

Le groupe Boc, par exemple, peut être introduit par réaction avec le di-t.butyldicarbonate en milieu basique. Le groupe Z peut être introduit selon la procédure générale décrite par E.C. Horning, Organic Synthesis, vol. III, Wiley, New York 1955, page 167.

Le composé de formule III ainsi obtenu est ensuite alkylé par réaction avec un halogénure de formule Hal-$CH_2$-COOR′, dans laquelle R′ et Hal sont tels que définis ci-dessus, en présence d'un agent de condensation basique, tel que l'hydroxyde ou le carbonate d'un métal alcalin, par exemple le carbonate de

potassium.

Le déblocage du groupe protecteur Y dans le composé de formule IV ainsi obtenu est effectué selon les méthodes décrites en littérature.

Par exemple, le groupe Boc peut être déplacé par traitement avec l'acide trifluoroacétique, seul ou en solution dans du chlorure de méthylène ou du chloroforme, avec de l'acide chlorhydrique gazeux dans de l'acétate d'éthyle, avec de l'acide chlorhydrique dans l'acide acétique ou avec de l'acide formique. Lorsque le déblocage est effectué avec l'acide trifluoroacétique, le produit de formule I est isolé sous forme de trifluoroacétate. Le sel ainsi obtenu peut être neutralisé avec une base, par exemple avec de l'hydroxyde d'ammonium et la base libérée peut être isolée ou directement transformée en un autre sel, par exemple le chlorhydrate ou le bromhydrate.

Le groupe Z peut être déplacé, par exemple, par hydrogénolyse.

Les composés ayant les formules III et IV ci-dessus sont nouveaux et représentent les intermédiaires clés du procédé de la présente invention.

Ainsi, la présente invention a pour objet, selon un autre de ses aspects, des composés de formule

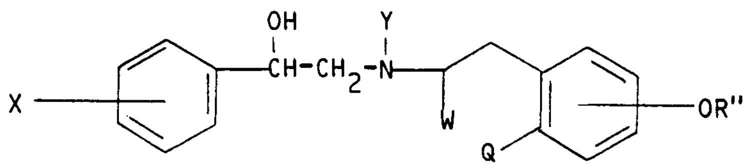

V

dans laquelle X, Y, W et Q sont tels que définis ci-dessus et R″ représente l'hydrogène ou un groupe $CH_2$-COOR′, R′ étant tel que défini ci-dessus.

Les réactions qui forment l'ensemble de ce procédé ne modifient pas la stéréochimie des composés concernés. Ainsi, le procédé de la présente invention permet d'opérer tant sur des mélanges racémiques que sur des isomères optiquement purs.

Les exemples suivants illustrent l'invention sans toutefois la limiter. Le symbole du pouvoir rotatoire est indiqué comme /alpha/, mais on doit l'entendre comme $[alpha]_D^{20}$ .

PREPARATION I

N-(7-hydroxy-1,2,3,4-tétrahydronapht-2-yl)-2-(3-chlorophényl)-2-hydroxyéthanamine; SR 58523, diastéréoisomère A.

On dissout dans 150 ml d'acétone 3,9 g de N-(7-hydroxy-1,2,3,4-tétrahydronapht-2-yl)-2-(3-chlorophényl)-2-hydroxyéthanamine sous forme de base, obtenue comme mélange 50/50 de diastéréoisomères selon l'exemple 8 du brevet européen 211721, et on le laisse cristalliser pendant une nuit après addition de quelques germes. On filtre le produit précipité et on évapore les eaux de filtration jusqu'à la moitié de leur volume. On les laisse dans le réfrigérateur pendant une nuit et l'on obtient encore un peu de produit pour un poids total de 1,8 g; p.f. 175 °C. On recristallise ce produit dans 120 ml d'acétone, on le laisse cristalliser pendant une nuit dans le réfrigérateur et l'on obtient 1,2 g de SR 58523; p.f. 182-184 °C, diastéréoisomère pur selon la RMN $^{13}C$ à 50 MHz. Sa configuration est (RS,SR).

PREPARATION II

N-(7-hydroxy-1,2,3,4-tétrahydronapht--2-yl)-2- (3- chlorophényl)-2-hydroxyéthanamine; SR 58524, diastéréoisomère B.

On utilise les eaux de filtration obtenues dans la PREPARATION I, on les évapore à sec et on dissout le résidu légèrement à chaud dans 25 ml d'acétate d'éthyle. Après filtration sur papier, on laisse cristalliser dans le réfrigérateur pendant une nuit, puis on filtre le produit et l'on obtient ainsi 1,2 g de SR 58524; p.f. 136-138 °C, mélange de diastéréoisomères (RR,SS)/(RS,SR) 75/25, selon la RMN $^{13}C$ à 50 MHz.

PREPARATION III

Monohydrate de R(+)-2-amino-7-hydroxytétraline; SR 58554.

A une solution dans 550 ml d'éthanol absolu de 50 g de 2-amino-7-méthoxytétraline base brute, obtenue du chlorhydrate correspondant (J. Chem. Soc., 1965, 2636-41) par neutralisation avec de l'hydroxyde de sodium à 10%, extraction avec de l'acétate d'éthyle et évaporation du solvant, on ajoute une solution de 43 g d'acide (+) mandélique dans 550 ml d'éthanol absolu. Après une nuit à la température ambiante, on filtre le précipité obtenu et on le cristallise deux fois dans l'éthanol absolu en récupérant chaque fois le produit cristallisé après repos d'une nuit à la température ambiante. On obtient ainsi 34,2 g (74%) de sel pur de l'acide (+) mandélique avec la (+)-2-amino-7-méthoxytétraline; p.f. 190-192 °C. Les eaux mères de cette première cristallisation sont séparées et utilisées pour la PREPARATION IV ci-dessous. On suspend 34 g du sel ainsi obtenu dans 300 ml d'eau et on rend basique le mélange réactionnel avec de l'hydroxyde de sodium N. On extrait la base avec de l'acétate d'éthyle, on évapore à sec et on reprend le résidu par 260 ml d'acide bromhydrique à 48%. On chauffe au reflux le mélange réactionnel pendant 3 h, on l'évapore à sec sous pression réduite et on reprend le résidu obtenu avec 70 ml d'eau. On rend basique la solution aqueuse avec de l'hydroxyde d'ammonium concentré, on la refroidit pendant une nuit et on la filtre. On obtient la R(+)-2-amino-7-hydroxytétraline (SR 58554) sous forme monohydratée; p.f. 143-144 °C, /alpha/ = +85,1 ° (méthanol, c = 0,5%).

Le chlorhydrate de ce produit a un pouvoir rotatoire qui correspond à celui de la littérature (Molecular Pharmacology, 1982, 22, 281-289).

PREPARATION IV

Monohydrate de S(-)-2-amino-7-hydroxytétraline, SR 58555.

On évapore à sec les eaux mères de la première cristallisation du produit SR 58554 (PREPARATION III), on suspend le résidu ainsi obtenu dans 300 ml d'eau et on rend la suspension basique par de l'hydroxyde de sodium N. On extrait la base avec de l'acétate d'éthyle. Suivant le mode opératoire décrit dans la PREPARATION III et en utilisant comme produits de départ la base ainsi obtenue et l'acide (-) mandélique, on obtient le sel de l'acide (-) mandélique avec la (-)-2-amino-7-méthoxytétraline (p.f. 189-191 °C) qui, par traitement avec l'hydroxyde de sodium N, deméthylation avec l'acide bromhydrique à 48%, et traitement avec de l'hydroxyde d'ammonium concentré, donne la S(-)-2-amino-7-hydroxytétraline, SR58555, sous forme monohydratée ; p.f.143-144 °C, /alpha/ = -86,9 ° (méthanol, c = 0,5%).

Le chlorhydrate de ce produit a un pouvoir rotatoire qui correspond à celui de la littérature (Molecular Pharmacology 1982, 22, 281-289).

PREPARATION V

N-[(2S)-7-hydroxy-1,2,3,4-tétrahydronapht-2-yl]-(R)-3-chloromandélamide, SR 58587.

On fait réagir sous courant d'azote 3,6 g d'acide (R)-3-chloromandélique (Bull. Soc. Chim. Fr., 1973, 12, 3330) avec 3,5 g de monohydrate de S(-)-2-amino-7-hydroxy-1,2,3,4-tétrahydronaphtalène (PREPARATION IV) et 7,7 g de BOP (hexafluorophosphate de benzotriazolyl-N-oxytris(diméthylamino)phosphonium) dans 60 ml de chlorure de méthylène anhydre. On ajoute 2,7 ml de triéthylamine, on laisse la solution obtenue sous agitation 5 heures à la température ambiante, on y ajoute 400 ml d'acétate d'éthyle et on lave avec une solution de bicarbonate de sodium, avec de l'eau, avec de l'acide chlorhydrique et enfin avec de l'eau. On sépare la phase organique, on l'évapore à sec, on traite le résidu ainsi obtenu avec 150 ml d'hydroxyde de sodium N et on extrait par de l'éther éthylique qui est ensuite éliminé. On acidifie la phase aqueuse avec de l'acide chlorhydrique concentré, on extrait par de l'acétate d'éthyle, on lave la phase organique à l'eau, on la sèche sur du sulfate de sodium, on la filtre et l'évapore à sec. L'huile ainsi obtenue est purifiée par flash chromatographie en utilisant comme éluant un mélange acétate d'éthyle/cyclohexane 70/30. On obtient le N-[(2S)-7-hydroxy-1,2,3,4-tétrahydronapht-2-yl]-(R)-3-chloromandélamide, sous forme d'une huile blanchâtre très épaisse qui est égrenée dans l'éther de pétrole; on obtient un solide ayant un point de fusion indéfini; SR 58587, /alpha/ = -95 ° (méthanol, c = 1%).

PREPARATION VI

N-[(2R)-7-hydroxy-1,2,3,4-tétrahydronapht-2-yl]-(S)-3-chloromandélamide, SR 58588.

Suivant le mode opératoire décrit dans la PREPARATION V et en remplaçant l'acide (R)-3-chloromandélique par l'acide (S)-3-chloromandélique (Bull. Soc. Chim. Fr., 1973, 12, 3330) et le monohydrate de S(-)-2-amino-7-hydroxy-1,2,3,4-tétrahydronaphtalène par le monohydrate de (R)-2-amino-7-hydroxy-1,2,3,4-tétrahydronaphtalène (PREPARATION III), on obtient le N-[(2R)-7-hydroxy-1,2,3,4-tétrahydronapht-2-yl]-(S)-3-chloromandélamide, sous forme d'un solide ayant un point de fusion indéfini; SR 58588, /alpha/ = +92,5° (méthanol, c = 1%).

PREPARATION VII

N-[(2R)-7-hydroxy-1,2,3,4-tétrahydronapht-2-yl]-(2S)-2-(3-chlorophényl)-2-hydroxyéthanamine, SR 58590.

A une solution de 3 g de N-[(2R)-7-hydroxy-1,2,3,4-tétrahydronapht-2-yl]-(S)-3-chloromandélamide, SR 58588, PREPARATION VI, dans 40 ml de tétrahydrofuranne anhydre, chauffée au reflux sous courant d'azote, on ajoute 2,7 ml d'une solution 10M de borane-méthylsulfure (réactif générant du diborane constitué par le complexe entre le borane et le diméthylsulfure) dans 20 ml de tétrahydrofuranne, et on continue le chauffage au reflux pendant 4 heures. A la solution refroidie à la température ambiante, on ajoute 25 ml de méthanol et on laisse sous agitation d'abord 30 minutes à la température ambiante et, ensuite, 30 minutes au reflux. On concentre sous pression réduite et, après filtration, on cristallise deux fois dans l'isopropanol. On obtient le N-[(2R)-7-hydroxy-1,2,3,4-tétrahydronapht-2-yl]-(2S)-2-(3-chlorophényl)-2-hydroxyéthanamine, SR 58590; p.f. 186-188°C; /alpha/ = + 76° (méthanol, c = 0,5%).

PREPARATION VIII

N-[(2S)-7-hydroxy-1,2,3,4-tétrahydronapht-2-yl]-(2R)-2-(3-chlorophényl)-2-hydroxyéthanamine, SR 58589.

Suivant le mode opératoire décrit dans la PREPARATION VII, en partant de 2,5 g de SR 58587 (PREPARATION V), on obtient le N-[(2S)-7-hydroxy-1,2,3,4-tétrahydronapht-2-yl]-(2R)-2-(3-chlorophényl)-2-hydroxyéthanamine, SR 58589; p.f. 185-187°C; /alpha/ = -78,5° (méthanol, c = 0,5%).

PREPARATION IX

N-[(2R)-7-hydroxy-1,2,3,4-tétrahydronapht-2-yl]-(R)-3-chloromandélamide, SR 58533.

A une suspension de 2,6 g de chlorhydrate de (R)-2-amino-7-hydroxytétraline,PREPARATION III, 2,4 g d'acide (R)-3-chloromandélique et 5,2 g de BOP dans 60 ml de chlorure de méthylène anhydre, on ajoute lentement 3,6 ml de triéthylamine, puis on laisse la solution obtenue sous agitation à la température ambiante pendant 3 heures. On ajoute 50 ml d'une solution saturée de chlorure de sodium, on laisse sous agitation pendant 30 min à la température ambiante, on ajoute 200 ml d'acétate d'éthyle et on sépare les phases. On lave la phase organique deux fois avec 30 ml d'acide chlorhydrique 2N, puis deux fois avec 30 ml d'une solution saturée de chlorure de sodium. On sèche la solution organique sur du sulfate de sodium, on la filtre et on l'évapore à sec. On purifie l'huile obtenue par flash chromatographie en utilisant comme solvant un mélange acétate d'éthyle/cyclohexane 55/45. On obtient un solide pâteux qu'on reprend avec 20 ml d'éther éthylique, dans lequel le produit cristallise. On ajoute 10 ml de cyclohexane, on filtre, on lave avec un mélange cyclohexane/éther éthylique 2/1 et on sèche sous pression réduite à 50°C. On obtient un stéréoisomère pur selon la RMN $^{13}C$ à 60 MHz; p.f. 132-134°C, /alpha/ = +24,9° (méthanol, c = 1%). Après une ultérieure purification par chromatographie, on obtient le N-[(2R)-7-hydroxy-1,2,3,4-tétrahydronapht-2-yl]-(R)-3-chloromandélamide énantiomériquement et chimiquement pur; SR 58533; p.f. 143-147°C; /alpha/ = +35,1° (méthanol, c = 1%).

PREPARATION X

N-[(2S)-7-hydroxy-1,2,3,4-tétrahydronapht-2-yl]-(S)-3-chloromandélamide, SR 58574.

Suivant le mode opératoire décrit dans la PREPARATION IX et en remplaçant l'acide (R)-3-chloromandélique par l'acide (S)-3-chloromandélique et le chlorhydrate de (R)-2-amino-7-hydroxytétraline par le chlorhydrate de (S)-2-amino-7-hydroxytétraline, PREPARATION IV, on obtient le N-[(2S)-7-hydroxy-1,2,3,4-tétrahydronapht-2-yl]-(S)-3-chloromandélamide; SR 58574; p.f. 145-146 °C; /alpha/ = -34,2 ° (méthanol, c = 1%).

PREPARATION XI

Chlorhydrate de la N-[(2R)-7-hydroxy-1,2,3,4-tétrahydronapht-2-yl]-(2R)-2-(3-chlorophényl)-2-hydroxyéthanamine, SR 58572 A.

En soumettant le produit obtenu dans la PREPARATION IX à une réduction avec borane-méthylsulfure comme décrit dans la PREPARATION VII, on obtient la N-[(2R)-7-hydroxy-1,2,3,4-tétrahydronapht-2-yl]-(2R)-2-(3-chlorophényl)-2-hydroxyéthanamine sous forme de base. Le produit ainsi obtenu est dissous dans de l'acétone, la solution filtrée est traitée avec de l'isopropanol saturé d'acide chlorhydrique. Après filtration, on cristallise deux fois dans l'isopropanol, on refroidit le produit, on le filtre et on le lave d'abord avec de l'isopropanol et ensuite avec de l'acétone. On obtient le chlorhydrate de N-[(2R)-7-hydroxy-1,2,3,4-tétrahydronapht-2-yl]-(2R)-2-(3-chlorophényl)-2-hydroxyéthanamine, SR 58572 A; p.f. 203-205 °C; /alpha/ = +36,4 ° (méthanol, c = 1%).

PREPARATION XII

Chlorhydrate de la N-[(2S)-7-hydroxy-1,2,3,4-tétrahydronapht-2-yl]-(2S)-2-(3-chlorophényl) 2-hydroxyéthanamine, SR 58575 A.

En soumettant le produit obtenu dans la PREPARATION X à une réduction avec borane-méthylsulfure comme décrit dans la PREPARATION VII, on obtient la N-[(2S)-7-hydroxy-1,2,3,4-tétrahydronapht-2-yl]-(2S)-2-(3-chlorophényl)-2-hydroxyéthanamine sous forme de base qui, en opérant comme décrit dans la PREPARATION XI, donne le chlorhydrate correspondant, SR 58575 A; p.f. 203-205 °C; /alpha/ = -35,8 ° (méthanol, c = 1%).

EXEMPLE 1

N-t.butyloxycarbonyl-N-(7-hydroxy-1,2,3,4-tétrahydronapht-2-yl)-2-(3-chlorophényl)-2-hydroxyéthanamine; diastéréoisomère A.

On dissout 1,5 g de SR 58523 obtenu comme décrit dans la PREPARATION I dans 100 ml de dioxanne et, à la solution obtenue, on ajoute, à la température ambiante, 5,1 ml d'hydroxyde de sodium N et 5,1 mM de di-t.butyldicarbonate. On agite le mélange pendant 1 heure à la température ambiante, 2 heures à 40 °C et encore une nuit à la température ambiante. Après évaporation sous pression réduite, on reprend le résidu par 150 ml d'éther éthylique, on le lave à l'eau et, après dessiccation, on évapore l'éther à sec. On analyse le résidu par RMN $^1$H et on confirme l'obtention du produit ci-dessus.

RMN $^1$H : ppm (DMSO D6) 1,32 (9H,s) 1,55-2,12 (2H,m) 2,25-282 (4H,m) 3,17 (2H,m) 3,45-3,90 (1H,m) 4,50-4,87 (1H,s, J=5Hz) 5,37 (1H,d,J=5Hz) 6,17-6,48 (2H,m) 6,57-6,82 (1H,m) 7,05-7,30 (4H,m) 8,80 (1H,s)

EXEMPLE 2

N-t.butyloxycarbonyl-N-(7-hydroxy-1,2,3,4-tétrahydronapht-2-yl)-2-(3-chlorophényl)-2-hydroxyéthanamine; diastéréoisomère B.

On dissout 1,5 g de SR 58524 obtenu comme décrit dans la PREPARATION II dans 100 ml de dioxanne et, à la solution obtenue, on ajoute, à la température ambiante, 5,1 ml d'hydroxyde de sodium N et 5,1 mM de di-t.butyldicarbonate. On agite le mélange pendant 1 heure à la température ambiante, 2

heures à 40 ° C et encore une nuit à la température ambiante. On évapore le mélange sous pression réduite et on reprend le résidu par 150 ml d'éther éthylique, on le lave à l'eau et, après dessiccation, on évapore l'éther à sec. On analyse le résidu par RMN $^1H$ et on confirme l'obtention du produit ci-dessus.

RMN $^1H$ : ppm (DMSO D6) 1,32 (9H,s) 1,55-2,12 (2H,m) 2,25-2,82 (4H,m) 3,17 (2H,m) 3,45-3,90 (1H,m) 4,50-4,87 (1H,s, J = 5Hz) 5,37 (1H,d, J = 5Hz) 6,17-6,48 (2H,m) 6,57-6,82 (1H,m) 7,05-7,30 (4H,m) 8,80 (1H,s)

EXEMPLE 3

Trifluoroacétate de N-(7-éthoxycarbonylméthoxy-1,2,3,4-tétrahydronapht-2-yl)-2-(3-chlorophényl)-2-hydroxyéthanamine; SR 58538 A, diastéréoisomère A.

(a) On dissout le produit obtenu dans l'exemple 1 dans 190 ml d'acétone et on ajoute à la solution 5,4 ml de bromoacétate d'éthyle et 6,36 g de carbonate de potassium. On agite le mélange au reflux pendant 6 heures, on le filtre, on l'évapore à sec, on reprend le résidu par de l'éther éthylique et on le filtre. Par évaporation du solvant, on obtient le N-t.butyloxycarbonyl-N-(7-éthoxycarbonylméthoxy-1,2,3,4-tétrahydronapht-2-yl)-2-(3-chlorophényl)-2-hydroxyéthanamine; diastéréoisomère A.

RMN $^1H$ : ppm (DMSO D6) 1,22 (3H,t,J = 7Hz) 1,35 (9H,s) 1,67-2,11 (2H,m) 2,52-2,98 (4H,m) 3,07-3,32 (2H,m) 3,55-3,87 (1H,m) 4,10 (2H,q,J = 7Hz) 4,51-4,87 (1H,m) 4,61 (2H,s) 5,25-5,62 (1H,m) 6,40-6,67 (2H,m) 6,77-7,02 (1H,m) 7,15-7,40 (4H,m)

(b) On dissout le produit ainsi obtenu dans 125 ml de chlorure de méthylène, on refroidit à 0-5 ° C et ensuite on ajoute à la solution 8 ml d'acide trifluoroacétique dissous dans 40 ml chlorure de méthylène. On agite le mélange 15 minutes à froid et 2 heures à la température ambiante. On évapore à sec à 40 ° C sous pression réduite, on cristallise le résidu dans l'éther éthylique et l'on obtient 3,9 g de produit fondant à 157-159 ° C. Le produit est cristallisé dans un mélange de 7,5 ml d'acétate d'éthyle et de 15 ml d'éther éthylique. On obtient ainsi 3,3 g de SR 58538 A, diastéréoisomère A; p.f. 156-158 ° C (rendement global à partir de SR 58523, 42%)

EXEMPLE 4

Trifluoroacétate de N-(7-éthoxycarbonylméthoxy-1,2,3,4-tétrahydronapht-2-yl)-2-(3-chlorophényl)-2-hydroxyéthanamine; SR 58539 A, diastéréoisomère B.

(a) On dissout le produit obtenu dans l'exemple 2 dans 200 ml d'acétone et on ajoute à la solution 5,6 ml de bromoacétate d'éthyle et 6,63 g de carbonate de potassium. On agite le mélange au reflux pendant 6 heures, on filtre, on évapore à sec, on reprend le résidu par de l'éther éthylique et on filtre. Par évaporation du solvant, on obtient la N-t.butyloxycarbonyl-N-(7-éthoxycarbonylméthoxy-1,2,3,4-tétrahydronapht-2-yl)-2-(3-chlorophényl)-2-hydroxyéthanamine; diastéréoisomère B.

RMN $^1H$ : ppm (DMSO D6) 1,22 (3H,t,J = 7Hz) 1,35 (9H,s) 1,67-2,11 (2H,m) 2,52-2,98 (4H,m) 3,07-3,32 (2H,m) 3,55-3,87 (1H,m) 4,10 (2H,q,J = 7Hz) 4,51-4,87 (1H,m) 4,61 (2H,s) 5,25-5,62 (1H,m) 6,40-6,67 (2H,m) 6,77-7,02 (1H,m) 7,15-7,40 (4H,m)

(b) On dissout le produit ainsi obtenu dans 130 ml de chlorure de méthylène, on refroidit à 0-5 ° C et ensuite on ajoute à la solution 12,7 ml d'acide trifluoroacétique dissous dans 40 ml de chlorure de méthylène. On agite le mélange 15 minutes à froid et 2 heures à la température ambiante. On évapore le mélange à sec à 40 ° C sous pression réduite, on cristallise le résidu dans l'éther éthylique et l'on obtient 5 g de produit fondant à 132-134 ° C. On dissout à chaud le produit obtenu dans 9 ml d'acétate d'éthyle, on ajoute à la solution 35 ml d'éther éthylique, on laisse refroidir et on filtre. On obtient ainsi 4,1 g de SR 58539 A; p.f. 134-136 ° C (rendement global à partir de SR 58524 : 49%).

EXEMPLE 5

Chlorhydrate de N-(7-éthoxycarbonylméthoxy-1,2,3,4-tétrahydronapht-2-yl)-2-(3-chlorophényl)-2-hydroxyéthanamine; SR 58539 B, diastéréoisomère B.

On suspend dans l'eau 7,5 g de SR 58539 A, préparé comme décrit dans l'exemple 4 avec 10 ml d'hydroxyde d'ammonium 15%. On extrait à l'éther, on lave à l'eau et, après évaporation de l'éther, on

dissout le résidu dans l'éthanol et on traite la solution avec une solution d'acide chlorhydrique dans l'éthanol. Après l'évaporation de l'éthanol, on reprend le résidu avec l'éther éthylique et on obtient un produit solide qu'on cristallise dans l'éthyl acétate. On obtient 3,1 g (50%) du produit ci-dessus; p.f. 137-139 ° C; diastéréoisomère pur selon la RMN $^{13}C$ à 50 MHz. Sa configuration est (RR,SS).

EXEMPLE 6

Chlorhydrate de N-(7-éthoxycarbonylméthoxy-1,2,3,4-tétrahydronapht-2-yl)-2-(3-chlorophényl)-2-hydroxyéthanamine; SR 58538 B, diastéréoisomère A.

On suspend dans l'eau 5,1 g de SR 58538 A, préparé comme décrit dans l'exemple 3 avec 7 ml d'hydroxyde d'ammonium 15%. On extrait à l'éther éthylique, on lave à l'eau et, après évaporation de l'éther, on dissout le résidu dans l'éthanol et on traite la solution avec une solution d'acide chlorhydrique dans l'éthanol. Après l'évaporation de l'éthanol, on reprend le résidu avec l'éther éthylique et on cristallise le produit obtenu dans un mélange éthanol/éther éthylique. On obtient 3,4 g (79%) du produit ci-dessus; p.f. 124-126 ° C, diastéréoisomère pur selon RMN $^{13}C$ à 50 MHz. Sa configuration est (RS,SR).

EXEMPLE 7

N-t.butyloxycarbonyl-N-(7-hydroxy-1,2,3,4-tétrahydronapht-2-yl)-2-(3-chlorophényl)-2-hydroxyéthanamine; diastéréoisomère A.

On dissout 4,9 g de SR 58523 obtenu comme décrit dans la PREPARATION I dans 200 ml de t.butanol et, à la solution obtenue, on ajoute, à la température ambiante, 16,9 ml d'hydroxyde de sodium N et 16,9 mM de di-t.butyldicarbonate. On agite le mélange pendant 1 heure à la température ambiante, 2 heures à 40 ° C et encore une nuit à la température ambiante. On reprend le mélange par 350 ml d'éther éthylique, on le lave à l'eau et, après dessiccation, on évapore l'éther à sec. On analyse le résidu par RMN $^{1}H$ et l'on confirme l'obtention du produit ci-dessus, identique au produit de l'exemple 1.

EXEMPLE 8

N-t.butyloxycarbonyl-N-(7-hydroxy-1,2,3,4-tétrahydronapht-2-yl)-2-(3-chlorophényl)-2-hydroxyéthanamine; diastéréoisomère B.

On dissout 5,1 g de SR 58524 obtenu comme décrit dans la PREPARATION II dans 200 ml de t.butanol et, à la solution obtenue, on ajoute, à la température ambiante, 17,6 ml d'hydroxyde de sodium N et 17,6 mM de di-t.butyldicarbonate. On agite le mélange pendant 1 heure à la température ambiante, 2 heures à 40 ° C et encore une nuit à la température ambiante. On reprend le mélange par 350 ml d'éther éthylique, on le lave à l'eau et, après dessiccation, on évapore l'éther à sec. On analyse le résidu par RMN $^{1}H$ et on confirme l'obtention du produit ci-dessus, identique au produit de l'exemple 2.

EXEMPLE 9

Trifluoroacétate de N-[2-(4-éthoxycarbonylméthoxyphényl)-1-méthyléthyl]-2-(3-chlorophényl)-2-hydroxyéthanamine.

(a) A un mélange de 1,6 g de N-[2-(4-hydroxyphényl)-1-méthyléthyl]-2-(3-chlorophényl)-2-hydroxyéthanamine (préparée comme décrit dans l'exemple 2 de EP 70134 mais en utilisant, comme agent réducteur, le cyanoborohydrure de sodium au lieu du borohydrure de sodium) et 5,5 ml d'hydroxyde de sodium N dans 50 ml de dioxanne, on ajoute goutte à goutte et à température ambiante 1,2 g de di-t.butyldicarbonate dissous dans 10 ml de dioxanne. On maintient ensuite l'agitation à 40 ° C pendant 2 heures, puis une nuit à température ambiante. Après dilution par l'éther éthylique, la solution organique est lavée 3 fois avec 20 ml d'eau et séchée sur du sulfate de sodium. La phase organique est concentrée sous pression réduite pour obtenir 2,5 g de N-t.butyloxycarbonyl-N-[2-(4-hydroxyphényl)-1-méthyléthyl]-2-(3-chlorophényl)-2-hydroxyéthanamine, sous forme d'un produit huileux qu'on utilise tel quel.

(b) On chauffe au reflux pendant 6 heures un mélange de 2,5 g du produit de l'étape (a), de 3,1 g de bromoacétate d'éthyle et de 2,5 mg de carbonate de potassium dans 80 ml d'acétone. Après refroidissement, le précipité de sels inorganiques est filtré et la solution évaporée sous pression réduite. Le résidu est purifié par chromatographie sur colonne de silice (éluant cyclohexane/acétate d'éthyle 7/3). On obtient 2 g de N-t.butyloxycarbonyl-N-[2-(4-éthoxycarbonylméthoxyphényl)-1-méthyléthyl]-2-(3-chlorophényl)-2-hydroxyéthanamine, sous forme de produit pur; huile jaune. Rendement 70%.

RMN ($CDCl_3$) 0,87-1,52 (15H,m) 2,32-2,82 (2H,m) 3,00-3,38 (2H,m) 3,70-4,25 (2H,m) 4,10 (2H,q,J = 7Hz) 4,42 (2H,s) 4,35-4,87 (2H,m) 6,52-7,00 (4H,m) 7,05 (3H,s) 7,22 (1H,s)

IR 1650-1690 $cm^{-1}$ et 1735-1760 $cm^{-1}$

(c) On ajoute à une solution de 1,5 g du produit de l'étape (b) dans 10 ml de chlorure de méthylène, à 0 °C, 10 ml d'acide trifluoroacétique. On agite 3 heures à la température ambiante puis on concentre sous pression réduite. Après lavage de l'huile résiduelle par l'éther éthylique, on obtient 1,4 g de trifluoroacétate de N-[2-(4-éthoxycarbonylméthoxyphényl)-1-méthyléthyl]-2-(3-chlorophényl)-2-hydroxyéthanamine sous forme d'huile jaune. Rendement 90%.

RMN $^1H$ : ppm (DMSO D6) 1,17 (3H,t,J = 7Hz) 1,07 (3H,d,J = 6Hz) 2,50-2,80 (1H,m) 2,82-3,60 (4H,m) 4,1 (2H,q,J = 7Hz) 4,65 (2H,s) 4,77-5,07 (1H,m) 5,12-5,92 (3H,m) 6,95 (4H,q, J = 8Hz) 7,3 (3H,s) 7,4 (1H,s)

IR 1670 $cm^{-1}$, 1750 $cm^{-1}$

Le produit ainsi obtenu peut être aisément saponifié avec la quantité calculée d'un agent alcalin et transformé dans l'acide libre correspondant.

EXEMPLE 10

On opère exactement comme décrit dans l'exemple 9, étape (a), puis

(b) on fait réagir le produit ainsi obtenu avec le bromoacétate de méthyle dans les conditions décrites dans l'exemple 9, étape (b), pour obtenir la N-t.butyloxycarbonyl-N-[2-(4-méthoxycarbonylméthoxyphényl)-1-méthyléthyl]-2-(3-chlorophényl)-2-hydroxyéthanamine; et

(c) on traite le produit ainsi obtenu avec l'acide trifluoroacétique dans les conditions décrites dans l'exemple 9, étape (c), pour obtenir le trifluoroacétate de N-[2-(4-méthoxycarbonylméthoxyphényl)-1-méthyléthyl]-2-(3-chlorophényl)-2-hydroxyéthanamine.

EXEMPLE 11

En opérant comme décrit dans l'exemple 6, par neutralisation du produit obtenu dans l'exemple 10 avec de l'hydroxyde d'ammonium et salification avec une solution d'acide bromhydrique au lieu de l'acide chlorhydrique dans l'éthanol, on obtient le bromhydrate de N-[2-(4-méthoxycarbonylméthoxyphényl)-1-méthyléthyl]-2-(3-chlorophényl)-2-hydroxyéthanamine.

EXEMPLE 12

Chlorhydrate de N-[(2S)-7-éthoxycarbonylméthoxy-1,2,3,4-tétrahydronapht-2-yl]-(2R)-2-(3-chlorophényl)-2-hydroxyéthanamine, SR 58611 A

(a) On dissout 1,8 g de SR 58589 obtenu comme décrit dans la PREPARATION VIII dans 12 ml de diméthylformamide et, après avoir ajouté 4 ml de triéthylamine, on laisse la solution ainsi obtenue sous agitation pendant 10 minutes à la température ambiante. On ajoute 1,36 g de di-t-butyldicarbonate et on laisse sous agitation le mélange réactionnel pendant 3 heures à la température ambiante. Après avoir ajouté 50 ml d'eau, on extrait 3 fois avec 70 ml d'éther éthylique. On lave la phase organique à l'eau, on sèche et on évapore à sec. On obtient 2,4 g (100%) de N-t.butyloxycarbonyl-N-[(2S)-7-hydroxy-1,2,3,4-tétrahydronapht-2-yl]-(2R)-2-(3-chlorophényl)-2-hydroxyéthanamine comme produit huileux.

(b) On dissout le produit ainsi obtenu dans 90 ml d'acétone et on ajoute 1,9 ml de bromoacétate d'éthyle et 2,34 g de carbonate de potassium anhydre. On chauffe au reflux pendant 6 heures le mélange réactionnel et, après filtration, on évapore l'acétone sous pression réduite. On dissout le résidu dans 20 ml de chlorure de méthylène, on refroidit à 0-5 °C et ensuite on ajoute à la solution 4,97 g d'acide trifluoroacétique dissous dans 10 ml de chlorure de méthylène. On laisse sous agitation le mélange réactionnel pendant 4 heures à la température ambiante et on le neutralise avec une solution de bicarbonate de sodium. Après avoir séparé la phase organique, on la lave à l'eau et on évapore à sec. On purifie le produit ainsi obtenu par chromatographie sur colonne de silice (éluant acétate d'éthyle). On dissout le produit ainsi obtenu dans l'éthanol et on le traite avec une solution d'acide chlorhydrique dans l'éthanol. Après évaporation du solvant, on obtient 0,55 g (22%) du chlorhydrate de N-[(2S)-7-éthoxycarbonylméthoxy-1,2,3,4-tétrahydronapht-2-yl]-(2R)-2-(3-chlorophényl)-2-hydroxyéthanamine comme solide vitreux; SR 58611 A, /alpha/ = -72,9° (c = 0,5%, méthanol).

| | |
|---|---|
| RMN $^1$H : ppm (DMSO D6) | 1,2 (3H,t,J=7Hz) 4,1 (3H,q,J=7Hz) 4,62 (2H,s) 5,0 (1H,m) |
| IR (KBr) : | 1775 $cm^{-1}$, 1612 $cm^{-1}$, 1203 $cm^{-1}$. |

In vitro, ce produit inhibe la motricité spontanée du côlon de rat (EP 255 415) avec une $CI_{50}$ de 3,5 (limites confidentielles : 2,6-4,7).$10^{-9}$M.

In vivo, dans le test de la motricité intestinale chez le rat anesthésié (EP 255 415), le SR 58611 A est actif avec une $DI_{50}$ de 13,0 (10-19) mcg/kg i.v.

En outre, ce produit est totalement inactif sur l'oreillette isolée de cobaye et très peu actif sur l'utérus isolé de rat.

EXEMPLE 13

Chlorhydrate de N-[(2R)-7-éthoxycarbonylméthoxy-1,2,3,4-tétrahydronapht-2-yl]-(2R)-2-(3-chlorophényl)-2-hydroxyéthanamine; SR 58612 A

(a) On suspend dans l'eau 2 g de SR 58572 A, PREPARATION XI, et, après avoir ajouté de l'hydroxyde d'ammonium concentré, on extrait par une solution à 10% v/v d'éthanol dans l'acétate d'éthyle, on évapore la phase organique séchée et on obtient 1,6 g de N-[(2R)-7-hydroxy-1,2,3,4-tétrahydronapht-2-yl]-(2R)-2-(3-chlorophényl)-2-hydroxyéthanamine base. En opérant comme décrit dans l'exemple 12 (a), on obtient 2,1 g (100%) de N-t.butyloxycarbonyl-N-[(2R)-7-hydroxy-1,2,3,4-tétrahydronapht-2-yl]-(2R)-2-(3-chlorophényl)-2-hydroxyéthanamine comme résidu huileux.

(b) En opérant comme décrit dans l'exemple 12 (b) et en partant du produit obtenu ci-dessus, on obtient 0,6 g (27%) du chlorhydrate de N-[(2R)-7-éthoxycarbonylméthoxy-1,2,3,4-tétrahydronapht-2-yl]-(2R)-2-(3-chlorophényl)-2-hydroxyéthanamine; SR 58612 A; p.f. 164-166°C; /alpha/ = +32,58° (c = 1%, méthanol).

In vitro, ce produit inhibe la motricité spontanée du côlon de rat (EP 255 415) avec une $CI_{50}$ de 2,7 (limites confidentielles : 2,0-3,5).$10^{-9}$M.

In vivo, dans le test de la motricité intestinale chez le rat anesthésié (EP 255 415), le SR 58612 A est actif avec une $DI_{50}$ de 7,7 (5,7-10,0) mcg/kg i.v.

En outre, ce produit est totalement inactif sur l'oreillette isolée de cobaye et peu actif sur l'utérus isolé de rat.

EXEMPLE 14

Chlorhydrate de N-[(2S)-7-éthoxycarbonylméthoxy-1,2,3,4-tétrahydronapht-2-yl]-(2S)-2-(3-chlorophényl)-2-hydroxyéthanamine; SR 58613 A

(a) On suspend dans l'eau 2 g de SR 58575 A, PREPARATION XII, et, après avoir ajouté de l'hydroxyde d'ammonium concentré, on extrait par une solution à 10% v/v d'éthanol dans l'acétate d'éthyle, on évapore le solvant et l'on obtient 1,6 g de N-[(2S)-7-hydroxy-1,2,3,4-tétrahydronapht-2-yl]-(2S)-2-(3-chlorophényl)-2-hydroxyéthanamine base. En opérant comme décrit dans l'exemple 12 (a), on obtient 2,1 g (100%) de N-t.butyloxycarbonyl-N-[(2S)-7-hydroxy-1,2,3,4-tétrahydronapht-2-yl]-(2S)-2-(3-chlorophényl)-2-hydroxyéthanamine comme résidu huileux.

(b) En opérant comme décrit dans l'exemple 12 (b) et en partant du produit obtenu ci-dessus, on obtient 0,4 g (18%) du chlorhydrate de N-[(2S)-7-éthoxycarbonylméthoxy-1,2,3,4-tétrahydronapht-2-yl]-(2S)-2-(3-chlorophényl)-2-hydroxyéthanamine; SR 58613 A; p.f. 164-166°C; /alpha/ = -34,3° (c = 1%, méthanol).

EXEMPLE 15

Chlorhydrate de N-[(2R)-7-méthoxycarbonylméthoxy-1,2,3,4-tétrahydronapht-2-yl]-(2R)-2-(3-chlorophényl)-2-hydroxyéthanamine; SR 58636 A

On dissout 4,6 g de N-t.butyloxycarbonyl-N-[(2R)-7-hydroxy-1,2,3,4-tétrahydronapht-2-yl]-(2R)-2-(3-chlorophényl)-2-hydroxyéthanamine obtenu comme décrit dans l'exemple 13 (a) dans 100 ml d'acétone et, après avoir ajouté 1,5 g de carbonate de potassium anhydre, on laisse au reflux pendant 1/2 heure. On refroidit le mélange réactionnel et on ajoute 5,3 g de carbonate de potassium anhydre, 0,9 g d'iodure de potassium et 5,9 g de bromoacétate de méthyle. On laisse le mélange réactionnel au reflux pendant 5

heures. Après filtration et évaporation à sec, on reprend le résidu par de l'éther éthylique et on lave à l'eau. Après avoir séché sur du sulfate de sodium, on évapore l'éther et on dissout le résidu dans 40 ml de chlorure de méthylène. On refroidit la solution ainsi obtenue à 0-5 °C et on y ajoute une solution froide de 20,7 g d'acide trifluoroacétique dans 30 ml de chlorure de méthylène. Le mélange réactionnel est mis sous agitation à 0 °C pendant 1/2 heure et à la température ambiante pendant 5 heures. On lave la solution avec du bicarbonate de sodium et ensuite avec de l'eau. Après avoir séché sur du sulfate de sodium, on évapore le solvant et on filtre avec de l'éther le résidu cristallisé. Après cristallisation dans l'acétate d'éthyle, on ajoute 50 ml d'eau et 10 ml d'hydroxyde d'ammonium. On extrait par de l'acétate d'éthyle, on sèche et on évapore à sec. On reprend le résidu par 10 ml d'acétate d'éthyle et on le rend acide par une solution d'acide chlorhydrique dans l'isopropanol. Le produit précipité est filtré et lavé avec une petite quantité d'acétate d'éthyle et d'éther. On obtient 0,7 g (15%) du chlorhydrate de N-[(2R)-7-méthoxycarbonylméthoxy-1,2,3,4-tétrahydronapht-2-yl]-(2R)-2-(3-chlorophényl)-2-hydroxyéthanamine; SR 58636 A; p.f. 152-154 °C; /alpha/ = +32,4 ° (c = 1%, méthanol).

## Revendications

**Revendications pour les Etats contractants suivants : AT, BE, CH, DE, FR, GB, GR, IT, LI, LU, NL, SE**

**1.** Procédé pour la préparation de composés de formule

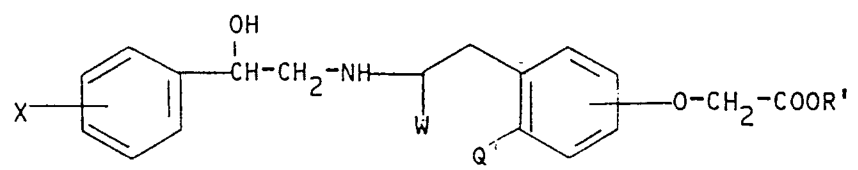

I

dans laquelle X représente l'hydrogène, un halogène, un groupe tri-fluorométhyle ou un groupe alkyle inférieur;W représente un groupe méthyle, Q représente l'hydrogène ou W et Q, ensemble, forment un groupe éthylène et R' représente un groupe alkyle inférieur et de leurs sels pharmaceutiquement acceptables, caractérisé en ce que

(a) on protège le groupe amino d'un composé de formule

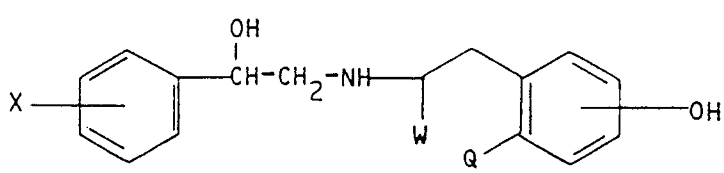

II

dans laquelle X, W et Q sont tels que définis ci-dessus;

(b) on soumet le composé ainsi obtenu de formule

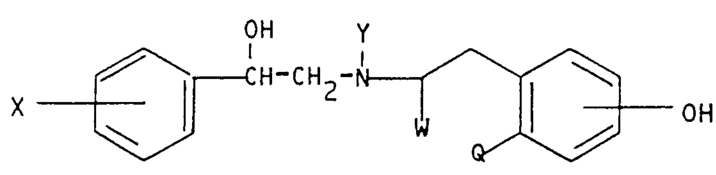

III

dans laquelle X, W et Q sont tels que définis ci-dessus et Y représente un groupe N-protecteur, à une alkylation avec un composé de formule Hal-$CH_2$-COOR', dans laquelle R' est tel que défini ci-dessus et Hal représente le chlore, le brome ou l'iode;

(c) on débloque le groupe amino protégé du composé ainsi obtenu de formule

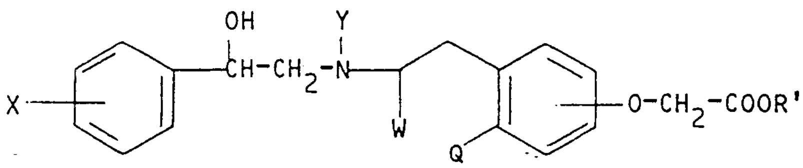

IV

où X, Y, W, Q et R' sont tels que définis ci-dessus;
et on transforme éventuellement le produit ainsi obtenu en l'un de ses sels pharmaceutiquement acceptables.

**2.** Procédé selon la revendication 1, caractérisé en ce que le groupe protecteur est le groupe benzyloxycarbonyle ou le groupe t.butyloxycarbonyle.

**3.** Procédé selon l'une des revendications 1 ou 2, caractérisé en ce que, comme composé de formule Hal-$CH_2$-COOR', on utilise le bromoacétate de méthyle ou d'éthyle.

**4.** Un composé de formule

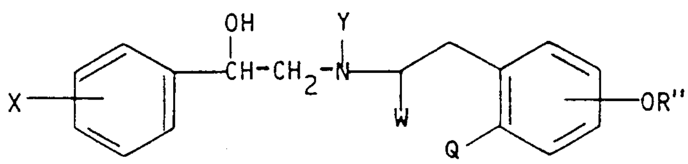

V

dans laquelle X représente l'hydrogène, un halogène, un groupe tri-fluorométhyle ou un groupe alkyle inférieur; W représente un groupe méthyle, Q représente l'hydrogène ou W et Q, ensemble, forment un groupe éthylène; R'' représente l'hydrogène ou un groupe $CH_2$-COOR', R' étant un groupe alkyle inférieur; et Y représente un groupe protecteur.

**5.** Composé selon la revendication 4, de formule V dans laquelle Y représente le groupe t.butyloxycarbonyle.

**6.** La N-t.butyloxycarbonyl-N-(7-hydroxy-1,2,3,4-tétrahydronapht-2-yl)-2-3-chlorophényl)-2-hydroxyéthanamine.

**7.** La N-t.butyloxycarbonyl-N-(7-éthoxycarbonylméthoxyl-1,2,3,4-tétrahydronapht-2-yl)-2-(3-chlorophényl)-2-hydroxyéthanamine.

**8.** La N-t.butyloxycarbonyl-N-[2-(4-hydroxyphényl)-1-méthyléthyl]-2-(3-chlorophényl)-2-hydroxyéthanamine.

**9.** La N-t.butyloxycarbonyl-N-[2-(4-éthoxycarbonylméthoxyphényl)-1-méthyléthyl]-2-(3-chlorophényl)-2-hydroxyéthanamine.

**10.** La N-t.butyloxycarbonyl-N-[2-(4-méthoxycarbonylméthoxyphényl)-1-méthyléthyl]-2-(3-chlorophényl)-2-hydroxyéthanamine.

**11.** La N-t.butyloxycarbonyl-N-[(2R)-7-hydroxy-1,2,3,4-tétrahydronapht-2-yl]-(2R)-2-(3-chlorophényl)-2-hydroxyéthanamine.

**12.** La N-t.butyloxycarbonyl-N-[(2S)-7-hydroxy-1,2,3,4-tétrahydronapht-2-yl)-(2R)-2-(3-chlorophényl)-2-hydroxyéthanamine.

**13.** La N-t.butyloxycarbonyl-N-[(2S)-7-hydroxy-1,2,3,4-tétrahydronapht-2-yl]-(2S)-2-(3-chlorophényl)-2-hydroxyéthanamine.

**14.** La N-[(2R)-7-éthoxycarbonylméthoxy-1,2,3,4-tétrahydronapht-2-yl]-(2R)-2-(3-chlorophényl)-2-hydroxyéthanamine et ses sels pharmaceutiquement acceptables.

**15.** La N-[(2S)-7-éthoxycarbonylméthoxy-1,2,3,4-tétrahydronapht-2-yl]-(2R)-2-(3-chlorophényl)-2-hydroxyéthanamine et ses sels pharmaceutiquement acceptables.

**16.** La N-[(2R)-7-méthoxycarbonylméthoxy-1,2,3,4-tétrahydronapht-2-yl]-(2R)-2-(3-chlorophényl)-2-hydroxyéthanamine et ses sels pharmaceutiquement acceptables.

**Revendications pour l'Etat contractant suivant : ES**

**1.** Procédé pour la préparation de composés de formule

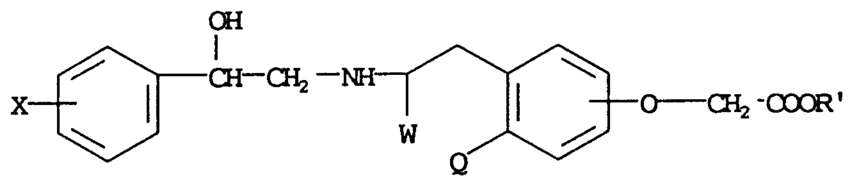

I

dans laquelle X représente l'hydrogène, un halogène, un groupe tri-fluorométhyle ou un groupe alkyle inférieur ; W représente un groupe méthyle, Q représente l'hydrogène ou W et Q, ensemble, forment un groupe éthylène et R' représente un groupe alkyle inférieur et de leurs sels pharmaceutiquement acceptables, caractérisé en ce que

(a) on protège le groupe amino d'un composé de formule

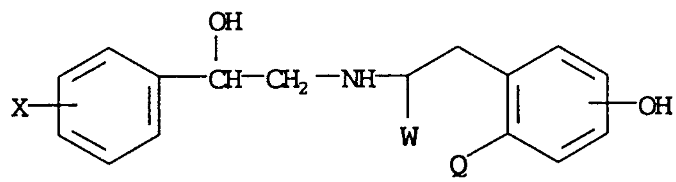

II

dans laquelle X, W et Q sont tels que définis ci-dessus;

(b) on soumet le composé ainsi obtenu de formule

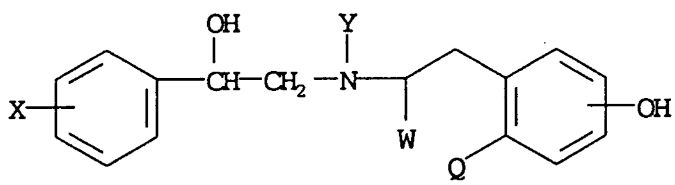

III

dans laquelle X, W et Q sont tels que définis ci-dessus et Y représente un groupe N-protecteur, à une alkylation avec un composé de formule Hal-$CH_2$-COOR', dans laquelle R' est tel que défini ci-dessus et Hal représente le chlore, le brome ou l'iode

(c) on débloque le groupe amino protégé du composé ainsi obtenu de formule

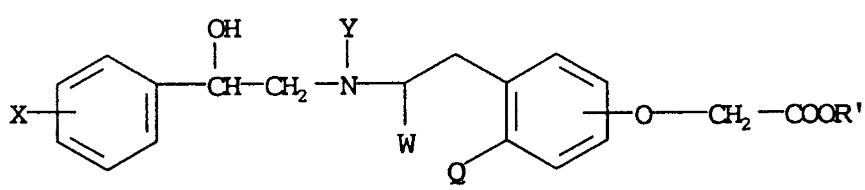

IV

où X, Y, W, Q et R' sont tels que définis ci-dessus;
et on transforme éventuellement le produit ainsi obtenu en l'un de ses sels pharmaceutiquement acceptables.

2. Procédé selon la revendication 1, caractérisé en ce que le groupe protecteur est le groupe benzyloxycarbonyle ou le groupe t.butyloxycarbonyle.

3. Procédé selon l'une des revendications 1 ou 2, caractérisé en ce que, comme composé de formule Hal-$CH_2$-COOR', on utilise le bromoacétate de méthyle ou d'éthyle.

4. Procédé pour l'obtention d'un composé de formule

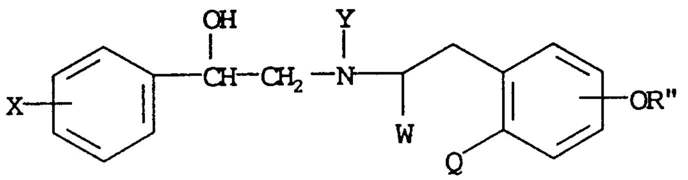

V

dans laquelle X représente l'hydrogène, un halogène, un groupe tri-fluorométhyle ou un groupe alkyle inférieur ; W représente un groupe méthyle, Q représente l'hydrogène ou W et Q, ensemble, forment un groupe éthylène ; R'' représente l'hydrogène ou un groupe $CH_2$-COOR', R' étant un groupe alkyle inférieur ; et Y représente un groupe protecteur, caractérisé en ce que :
(a) on protège le groupe amino d'un composé de formule

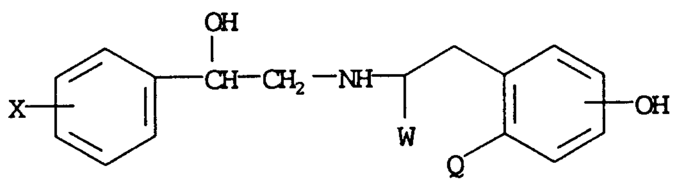

II

dans laquelle X, W et Q sont tels que définis ci-dessus;
(b) on soumet éventuellement le composé ainsi obtenu de formule

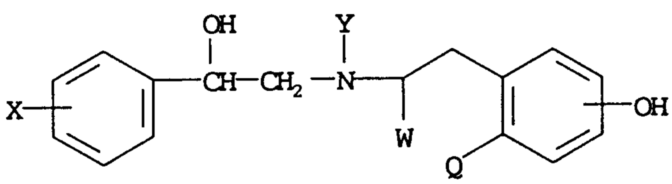

III

dans laquelle X, W et Q sont tels que définis ci-dessus et Y représente un groupe N-protecteur, à une alkylation avec un composé de formule Hal-$CH_2$-COOR', dans laquelle R' est tel que défini ci-dessus et Hal représente le chlore, le brome ou l'iode pour former le composé de formule :

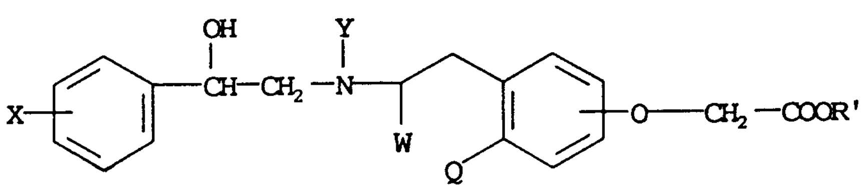

IV

5. Procédé selon la revendication 4, caractérisé en ce que l'on protége le groupe amino par réaction avec le di-t.butyldicarbonate en milieu basique.

6. Procédé selon l'une des revendications 4 ou 5, caractérisé en ce que l'on utilise la N-(7-hydroxy-1,2,3,4-tétrahydronapht-2-yl)-2-(3-chlorophényl)-2-hydroxyéthanamine comme composé de formule II pour former la N-t.butyloxycarbonyl-N-(7-hydroxy-1,2,3,4-tétrahydronapht-2-yl)-2-(3-chlorophényl)-2-hydroxyéthanamine.

7. Procédé selon l'une des revendications 4 ou 5, caractérisé en ce que l'on utilise la N-(7-hydroxy-1,2,3,4-tétrahydronapht-2-yl)-2-(3-chlorophényl)-2-hydroxyéthanamine comme composé de formule II et un composé de formule Hal-$CH_2$-COOR' dans laquelle R' est le groupe éthyle, pour former la N-t.butyloxycarbonyl-N-(7-éthoxycarbonylméthoxy-1,2,3,4-tétrahydronapht-2-yl)-2-(3-chlorophényl)-2-hydroxyéthanamine.

8. Procédé selon l'une des revendications 4 ou 5, caractérisé en ce que l'on utilise la N-[2-(4-hydroxyphényl)-1-méthyléthyl]-2-(3-chlorophényl)-2-hydroxyéthanamine comme composé de formule II pour former la N-t.butyloxycarbonyl-N-[2-(4-hydroxyphényl)-1-méthyléthyl]-2-(3-chlorophényl)-2-hydroxyéthanamine.

9. Procédé selon l'une des revendications 4 ou 5, caractérisé en ce que l'on utilise la N-[2-(4-hydroxyphényl)-1-méthyléthyl]-2-(3-chlorophényl)-2-hydroxyéthanamine comme composé de formule II et un composé de formule Hal-$CH_2$-COOR' dans laquelle R' est le groupe éthyle pour former la N-t.butyloxycarbonyl-N-[2-(4-éthoxycarbonylméthoxyphényl)-1-méthyléthyl]-2-(3-chlorophényl)-2-hydroxyéthanamine.

10. Procédé selon l'une des revendications 4 ou 5, caractérisé en ce que l'on utilise la N-[2-(4-hydroxyphényl)-1-méthyléthyl]-2-(3-chlorophényl)-2-hydroxyéthanamine comme composé de formule II et un composé de formule Hal-$CH_2$-COOR' dans laquelle R' est le groupe méthyle pour former la N-t.butyloxycarbonyl-N-[2-(4-méthoxycarbonylméthoxyphényl)-1-méthyléthyl]-2-(3-chlorophényl)-2-hydroxyéthanamine.

11. Procédé selon l'une des revendications 4 ou 5, caractérisé en ce que l'on utilise la N-[(2R)-7-hydroxy-1,2,3,4-tétrahydronapht-2-yl]-(2R)-2-(3-chlorophényl)-2-hydroxyéthanamine comme composé de formule II pour former la N-t.butyloxycarbonyl-N-[(2R)-7-hydroxy-1,2,3,4-tétrahydronapht-2-yl]-(2R)-2-(3-chlorophényl)-2-hydroxyéthanamine.

12. Procédé selon l'une des revendications 4 ou 5, caractérisé en ce que l'on utilise la N-[(2S)-7-hydroxy-1,2,3,4-tétrahydronapht-2-yl]-(2R)-2-(3-chlorophényl)-2-hydroxyéthanamine comme composé de formule II pour former la N-t.butyloxycarbonyl-N-[(2S)-7-hydroxy-1,2,3,4-tétrahydronapht-2-yl]-(2R)-2-(3-chlorophényl)-2-hydroxyéthanamine.

13. Procédé selon l'une des revendications 4 ou 5, caractérisé en ce que l'on utilise la N-[(2S)-7-hydroxy-1,2,3,4-tétrahydronapht-2-yl]-(2S)-2-(3-chlorophényl)-2-hydroxyéthanamine comme composé de formule II pour former la N-t.butyloxycarbonyl-N-[(2S)-7-hydroxy-1,2,3,4-tétrahydronapht-2-yl]-(2S)-2-(3-chlorophényl)-2-hydroxyéthanamine.

14. Procédé selon la revendication 1 caractérisé en ce que l'on utilise la N-[(2R)-7-hydroxy-1,2,3,4-tétrahydronapht-2-yl]-(2R)-2-(3-chlorophényl)-2-hydroxyéthanamine comme composé de formule II et un composé de formule Hal-$CH_2$-COOR' dans laquelle R' est le groupe éthyle pour former la N-[(2R)-7-éthoxycarbonylméthoxy-1,2,3,4-tétrahydronapht-2-yl]-(2R)-2-(3-chlorophényl)-2-hydroxyéthanamine et ses sels pharmaceutiquement acceptables.

15. Procédé selon la revendication 1 caractérisé en ce que l'on utilise la N-[(2S)-7-hydroxy-1,2,3,4-tétrahydronapht-2-yl]-(2R)-2-(3-chlorophényl)-2-hydroxyéthanamine comme composé de formule II et un composé de formule Hal-$CH_2$-COOR' dans laquelle R' est le groupe éthyle pour former la N-[(2S)-7-éthoxycarbonylméthoxy-1,2,3,4-tétrahydronapht-2-yl]-(2R)-2-(3-chlorophényl)-2-hydroxyéthanamine et ses sels pharmaceutiquement acceptables.

16. Procédé selon la revendication 1, caractérisé en ce que l'on utilise la N-[(2R)-7-hydroxy-1,2,3,4-tétrahydronapht-2-yl]-(2R)-2-(3-chlorophényl)-2-hydroxyéthanamine comme composé de formule II et un composé de formule Hal-$CH_2$-COOR' dans laquelle R' est le groupe méthyle pour former la N-[(2R)-7-méthoxycarbonylméthoxy-1,2,3,4-tétrahydronapht-2-yl]-(2R)-2-(3-chlorophényl)-2-hydroxyéthanamine et ses sels pharmaceutiquement acceptables.

**Claims**

**Claims for the following Contracting States : AT, BE, CH, DE, FR, GB, GR, IT, LI, LU, NL, SE**

**1.** Process for the preparation of compounds of formula :

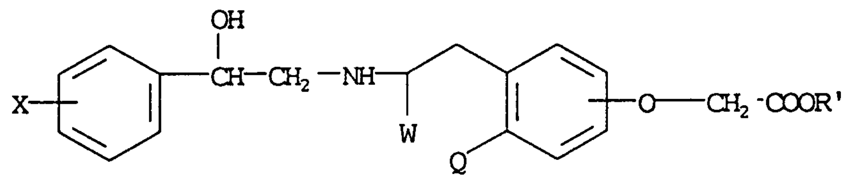 I

wherein X is hydrogen, halogen, a trifluoromethyl group or a lower alkyl group ; W represents a methyl group, Q represents hydrogen or W and Q, together, form an ethylene group and R' represents a lower alkyl group and of their pharmaceutically acceptable salts, which comprises :

(a) protecting the amino group of a compound of formula :

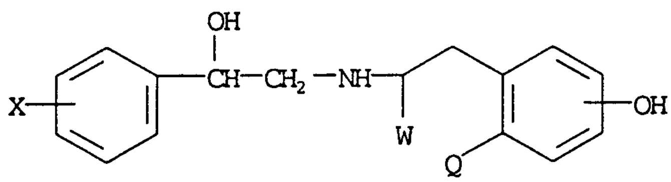 II

wherein X, W and Q are as defined hereinabove ;

(b)submitting the compound thus obtained of formula :

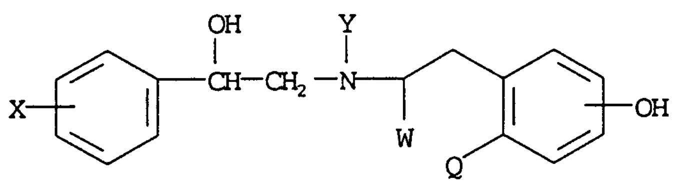 III

wherein X, W and Q are as defined hereinabove and Y is a N-protecting group, to an alkylation with a compound of formula Hal-$CH_2$-COOR', wherein R' is as defined hereinabove and Hal is chlorine, bromine or iodine ;

(c) deblocking the protected amino group of the compound thus obtained of formula :

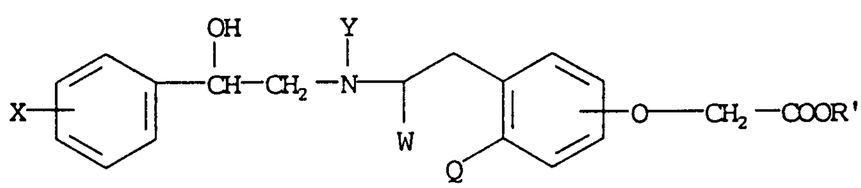 IV

wherein X, Y, W, Q and R' are as defined hereinabove ;

and optionally converting the product thus obtained into one of its pharmaceutically acceptable salts.

**2.** Process according to claim 1, characterized in that the protecting group is the benzyloxycarbonyl group or the t.butyloxycarbonyl group.

3. Process according to one of claims 1 or 2, characterized in that the methyl or ethyl bromoacetate is used as compound of formula Hal-$CH_2$-COOR'.

4. A compound of formula :

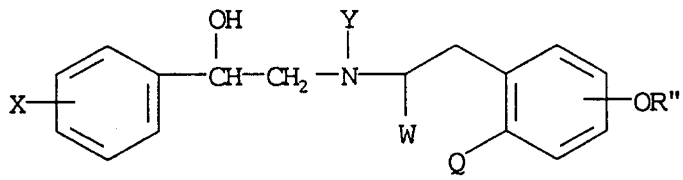

V

wherein X is hydrogen, halogen, a trifluoromethyl group or a lower alkyl group ; W is a methyl group, Q is hydrogen or W and Q, together , form an ethylene group ; R" is hydrogen or a $CH_2$-COOR' group, wherein R' is a lower alkyl group ; and Y is a protecting group.

5. Compound according to claim 4 of formula V in which Y is the t.butyloxycarbonyl group.

6. N-t.butyloxycarbonyl-N-(7-hydroxy-1,2,3,4-tetrahydronapht-2-yl)-2-(3-chlorophenyl)-2-hydroxyethanamine.

7. N-t.butyloxycarbonyl-N-(7-ethoxycarbonylmethoxy-1,2,3,4-tetrahydronapht-2-yl)-2-(3-chlorophenyl)-2-hydroxyethanamine.

8. N-t.butyloxycarbonyl-N-[2-(4-hydroxyphenyl)-1-methylethyl]-2-(3-chlorophenyl)-2-hydroxyethanamine.

9. N-t.butyloxycarbonyl-N-[2-(4-ethoxycarbonylmethoxyphenyl)-1-methylethyl]-2-(3-chlorophenyl)-2-hydroxyethanamine.

10. N-t.butyloxycarbonyl-N-[2-(4-methoxycarbonylmethoxyphenyl)-1-methylethyl]-2-(3-chlorophenyl)-2-hydroxyethanamine.

11. N-t.butyloxycarbonyl-N-[(2R)-7-hydroxy-1,2,3,4-tetrahydronapht-2-yl]-(2R)-2-(3-chlorophenyl)-2-hydroxyethanamine.

12. N-t.butyloxycarbonyl-N-[(2S)-7-hydroxy-1,2,3,4-tetrahydronapht-2-yl]-(2R)-2-(3-chlorophenyl)-2-hydroxyethanamine.

13. N-t.butyloxycarbonyl-N-[(2S)-7-hydroxy-1,2,3,4-tetrahydronapht-2-yl]-(2S)-2-(3-chlorophenyl)-2-hydroxyethanamine.

14. N-[(2R)-7-ethoxycarbonylmethoxy-1,2,3,4-tetrahydronapht-2-yl]-(2R)-2-(3-chlorophenyl)-2-hydroxyethanamine and its pharmaceutically acceptable salts.

15. N-[(2S)-7-ethoxycarbonylmethoxy-1,2,3,4-tetrahydronapht-2-yl]-(2R)-2-(3-chlorophenyl)-2-hydroxyethanamine and its pharmaceutically acceptable salts.

16. N-[(2R)-7-methoxycarbonylmethoxy-1,2,3,4-tetrahydronapht-2-yl]-(2R)-2-(3-chlorophenyl)-2-hydroxyethanamine and its pharmaceutically acceptable salts.

**Claims for the following Contracting State : ES**

1. Process for the preparation of compounds of formula :

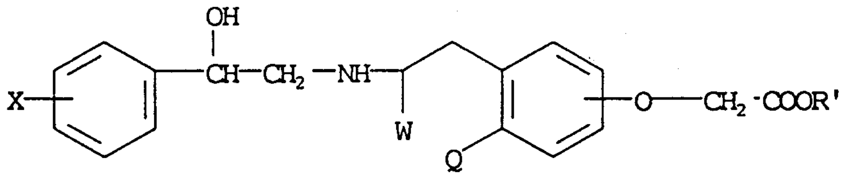 I

wherein X is hydrogen, halogen, a trifluoromethyl group or a lower alkyl group ; W represents a methyl group, Q represents hydrogen or W and Q, together, form an ethylene group and R' represents a lower alkyl group and of their pharmaceutically acceptable salts, which comprises:

(a) protecting the amino group of a compound of formula :

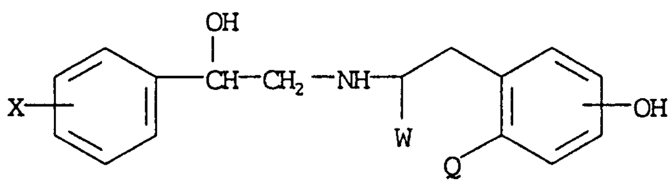 II

wherein X, W and Q are as defined hereinabove ;

(b)submitting the compound thus obtained of formula :

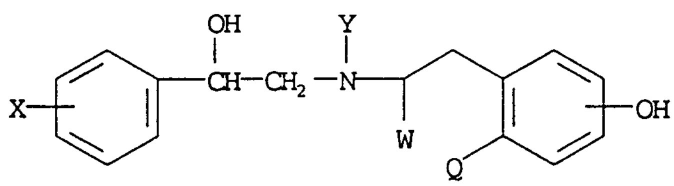 III

wherein X, W and Q are as defined hereinabove and Y is a N-protecting group, to an alkylation with a compound of formula $Hal-CH_2-COOR'$, wherein R' is as defined hereinabove and Hal is chlorine, bromine or iodine;

(c) deblocking the protected amino group of the compound thus obtained of formula :

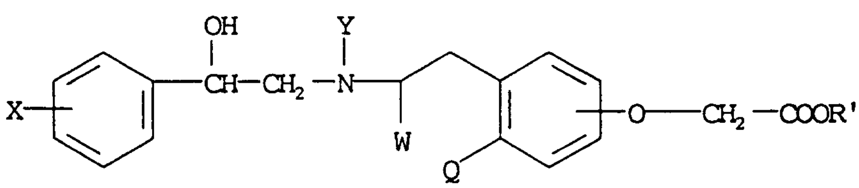 IV

wherein X, Y, W, Q and R' are as defined hereinabove ;

and optionally converting the product thus obtained into one of its pharmaceutically acceptable salts.

2. Process according to claim 1, characterized in that the protecting group is the benzyloxycarbonyl group or the t.butyloxycarbonyl group.

3. Process according to one of claims 1 or 2, characterized in that the methyl or ethyl bromoacetate is used as compound of formula $Hal-CH_2-COOR'$.

**4.** Process for the obtention of a compound of formula :

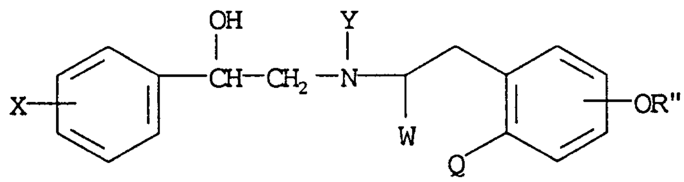

V

wherein X is hydrogen, halogen, a trifluoromethyl group or a lower alkyl group ; W is a methyl group, Q is hydrogen or W and Q, together, form an ethylene group ; R" is hydrogen or a $CH_2$-COOR' group, wherein R' is a lower alkyl group ; and Y is a protecting group, characterized in that it comprises :

(a) protecting the amino group of a compound of formula :

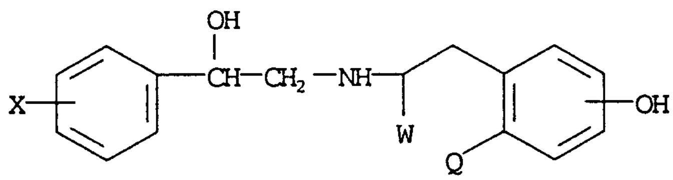

II

wherein X, W and Q are as defined hereinabove ;

(b) optionally submitting the compound thus obtained of formula :

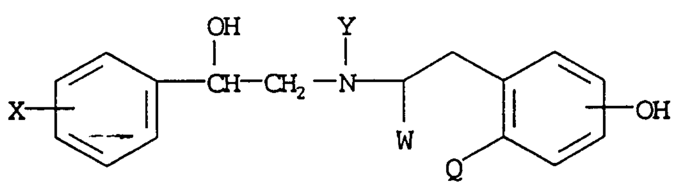

III

wherein X, W and Q are as defined hereinabove and Y is a N-protecting group, to an alkylation with a compound of formula Hal-$CH_2$-COOR', wherein R' is as defined hereinabove and Hal is chlorine, bromine or iodine, in order to form a compound of formula :

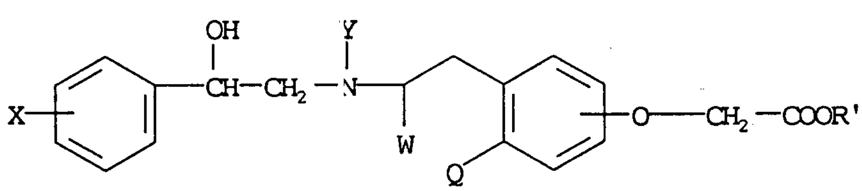

IV

**5.** Process according to claim 4, characterized in that the amino group is protected by reaction with the di-t.butyldicarbonate in a basic medium.

**6.** Process according to one of claims 4 or 5, characterized in that the N-(7-hydroxy-1,2,3,4-tetrahydronapht-2-yl)-2-(3-chlorophenyl)-2-hydroxyethanamine is used as compound of formula II to form the N-t.butyloxycarbonyl-N-(7-hydroxy-1,2,3,4-tetrahydronap ht-2-yl)-2-(3-chlorophenyl)-2-hydroxyethanamine.

**7.** Process according to one of claims 4 or 5, characterized in that the N-(7-hydroxy-1,2,3,4-tetrahydronapht-2-yl)-2-(3-chlorophenyl)-2-hydroxyethanamine as compound of formula II and a compound of formula Hal-$CH_2$-COOR' in which R' is an ethyl group are used to form the N-t.butyloxycarbonyl-N-(7-ethoxycarbonylmethoxy-1,2,3,4-tetrahydronapht-2-yl)-2-(3-chlorophenyl)-2-hydroxyethanamine.

8. Process according to one of claims 4 or 5, characterized in that the N-[2-(4-hydroxyphenyl)-1-methylethyl]-2-(3-chlorophenyl)-2-hydroxyethanamine is used as compound of formula II to form the N-t.butyloxycarbonyl-N-[2-(4-hydroxyphenyl)-1-methylethyl]-2-(3-chlorophenyl)-2-hydroxyethanamine.

9. Process according to one of claims 4 or 5, characterized in that the N-[2-(4-hydroxyphenyl)-1-methylethyl]-2-(3-chlorophenyl)-2-hydroxyethanamine as compound of formula II and a compound of formula Hal-$CH_2$-COOR' in which R is an ethyl group are used to form the N-t.butyloxycarbonyl-N-[2-(4-ethoxycarbonylmethoxyphenyl)-1-methylethyl]-2-(3-chlorophenyl)-2-hydroxyethanamine.

10. Process according to one of claims 4 or 5, characterized in that the N-[2-(4-hydroxyphenyl)-1-methylethyl]-2-(3-chlorophenyl)-2-hydroxyethanamine as compound of formula II and a compound of formula Hal-$CH_2$-COOR' in which R is a methyl group are used to form the N-t.butyloxycarbonyl-N-[2-(4-methoxycarbonylmethoxyphenyl)-1-methylethyl]-2-(3-chlorophenyl)-2-hydroxyethanamine.

11. Process according to one of claims 4 or 5, characterized in that the N-[(2R)-7-hydroxy-1,2,3,4-tetrahydronapht-2-yl]-(2R)-2-(3-chlorophenyl)-2-hydroxyethanamine is used as compound of formula II to form the N-t.butyloxycarbonyl-N-[(2R)-7-hydroxy-1,2,3, 4-tetrahydronapht-2-yl]-(2R)-2-(3-chlorophenyl)-2-hydroxyethanamine.

12. Process according to one of claims 4 or 5, characterized in that the N-[(2S)-7-hydroxy-1,2,3,4-tetrahydronapht-2-yl]-(2R)-2-(3-chlorophenyl)-2-hydroxyethanamine is used as compound of formula II to form the N-t.butyloxycarbonyl-N-[(2S)-7-hydroxy-1,2,3, 4-tetrahydronapht-2-yl]-(2R)-2-(3-chlorophenyl)-2-hydroxyethanamine.

13. Process according to one of claims 4 or 5, characterized in that the N-[(2S)-7-hydroxy-1,2,3,4-tetrahydronapht-2-yl]-(2S)-2-(3-chlorophenyl)-2-hydroxyethanamine is used as compound of formula II to form the N-t.butyloxycarbonyl-N-[(2S)-7-hydroxy-1,2,3, 4-tetrahydronapht-2-yl]-(2S)-2-(3-chlorophenyl)-2-hydroxyethanamine.

14. Process according to one of claims 4 or 5, characterized in that the N-[(2R)-7-hydroxy-1,2,3,4-tetrahydronapht-2-yl]-(2R)-2-(3-chlorophenyl)-2-hydroxyethanamine as compound of formula II and a compound of formula Hal-$CH_2$-COOR' in which R' is an ethyl group are used to form the N-[(2R)-7-ethoxycarbonylmethoxy-1,2,3,4-tetrahydronapht-2-yl]-(2R)-2-(3-chlorophenyl)-2-hydroxyethanamine and its pharmaceutically acceptable salts.

15. Process according to one of claims 4 or 5, characterized in that the N-[(2S)-7-hydroxy-1,2,3,4-tetrahydronapht-2-yl]-(2R)-2-(3-chlorophenyl)-2-hydroxyethanamine as compound of formula II and a compound of formula Hal-$CH_2$-COOR' in which R' is an ethyl group are used to form the N-[(2S)-7-ethoxycarbonylmethoxy-1,2,3,4-tetrahydronapht-2-yl]-(2R)-2-(3-chlorophenyl)-2-hydroxyethanamine and its pharmaceutically acceptable salts.

16. Process according to claim 1, characterized in that the N-[(2R)-7-hydroxy-1,2,3,4-tetrahydronapht-2-yl]-(2R)-2-(3-chlorophenyl)-2-hydroxyethanamine as compound of formula II and a compound of a formula Hal-$CH_2$-COOR' in which R' is a methyl group are used to form the N-[(2R)-7-methoxycarbonylmethoxy-1,2,3,4-tetrahydronapht-2-yl]-(2R)-2-(3-chlorophenyl)-2-hydroxyethanamine and its pharmaceutically acceptable salts.

**Patentansprüche**

**Patentansprüche für folgende Vertragsstaaten : AT, BE, CH, DE, FR, GB, GR, IT, LI, LU, NL, SE**

1. Verfahren zur Herstellung von Verbindungen der Formel

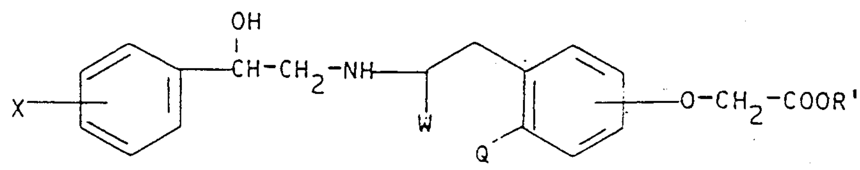

I,

worin X Wasserstoff, ein Halogen, eine Trifluormethylgruppe oder eine nied.Alkylgruppe darstellt, W eine Methylgruppe darstellt, Q Wasserstoff darstellt, oder W und Q zusammen eine Ethylengruppe bilden und R' eine nied.Alkylgruppe darstellt, sowie deren pharmazeutisch akzeptablen Salzen, dadurch gekennzeichnet, daß

(a) die Aminogruppe einer Verbindung der Formel

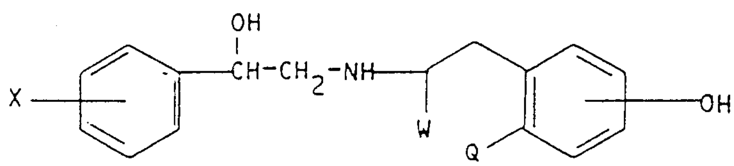

II,

worin X, W und Q wie oben definiert sind, geschützt wird;

(b) die so erhaltene Verbindung der Formel

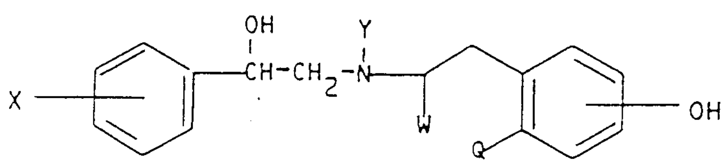

III,

worin X, W und Q wie oben definiert sind und Y eine N-Schutzgruppe darstellt, einer Alkylierung mit einer Verbindung der Formel Hal-$CH_2$-COOR' unterzogen wird, worin R' wie oben definiert ist und Hai für Chlor, Brom oder lod steht;

c) die geschützte Aminogruppe der so erhaltenen Verbindung der Formel

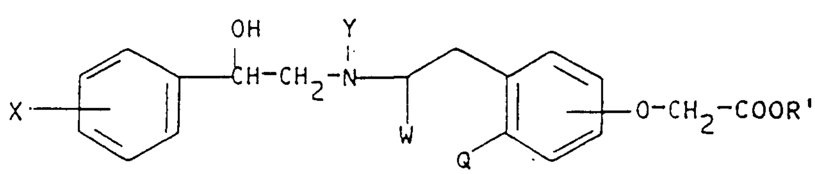

IV,

worin X, Y, W, Q und R' wie oben definiert sind, entschützt wird,

und das so erhaltene Produkt gegebenenfalls in eines seiner pharmazeutisch akzeptablen Salze übergeführt wird.

2. Verfahren nach Anspruch 1, dadurch gekennzeichnet, daß die Schutzgruppe Benzyloxycarbonyl oder tert.Butyloxycarbonyal ist.

3. Verfahren nach einem der Ansprüche 1 oder 2, dadurch gekennzeichnet, daß als Verbindung der Formel Hal-$CH_2$-COOR' Methyl- oder Ethylbromacetat verwendet wird.

4. Verbindung der Formel

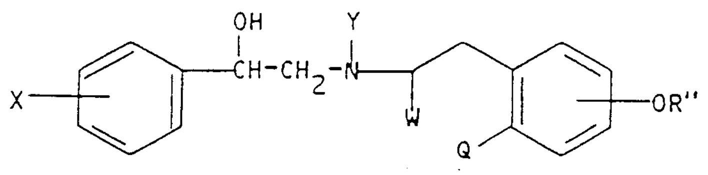

V,

worin X Wasserstoff, ein Halogen, eine Trifluormethylgruppe oder eine nied.Alkylgruppe darstellt, W eine Methylgruppe darstellt, Q Wasserstoff darstellt, oder W und Q zusammen eine Ethylengruppe bilden, R'' Wasserstoff oder eine Gruppe $CH_2$-COOR' darstellt, wobei R' eine nied.Alkylgruppe ist, und Y eine Schutzgruppe darstellt.

5. Verbindung nach Anspruch 4 der Formel V, worin Y tert.Butyloxycarbonyl darstellt.

6. N-tert.Butyloxycarbonyl-N-(7-hydroxy-1,2,3,4,-tetrahydronaphth-2-yl)-2-(3-chlorphenyl)-2-hydroxyethanamin.

7. N-tert.Butyloxycarbonyl-N-(7-ethoxycarbonylmethoxy-1,2,3,4-tetrahydronaphth-2-yl)-2-(3-chlorphenyl)-2-hydroxyethanamin.

8. N-tert.Butyloxycarbonyl-N-[2-(4-hydroxyphenyl)-1-methylethyl]-2-(3-chlorphenyl)-2-hydroxyethanamin.

9. N-tert.Butyloxycarbonyl-N-[2-(4-ethoxycarbonylmethoxyphenyl)-1-methylethyl]-2-(3-chlorphenyl)-2-hydroxyethanamin.

10. N-tert.Butyloxycarbonyl-N-[2-(4-methoxycarbonylmethoxyphenyl)-1-methylethyl]-2-(3-chlorphenyl)-2-hydroxyethanamin.

11. N-tert.Butyloxycarbonyl-N-[(2R)-7-hydroxy-1,2,3,4-tetrahydronaphth-2-yl]-(2R)-2-(3-chlorphenyl)-2-hydroxyethanamin.

12. N-tert.Butyloxycarbonyl-N-[(2S)-7-hydroxy-1,2,3,4-tetrahydronaphth-2-yl]-(2R)-2-(3-chlorphenyl)-2-hydroxyethanamin.

13. N-tert.Butyloxycarbonyl-N-[(2S)-7-hydroxy-1,2,3,4-tetrahydronaphth-2-yl]-(2S)-2-(3-chlorphenyl)-2-hydroxyethanamin.

14. N-[(2R)-7-Ethoxycarbonylmethoxy-1,2,3,4-tetrahydronaphth-2-yl]-(2R)-2-(3-chlorphenyl)-2-hydroxyethanamin und dessen pharmazeutisch akzeptable Salze.

15. N-[(2S)-7-Ethoxycarbonylmethoxy-1,2,3,4-tetrahydronaphth-2-yl]-(2R)-2-(3-chlorphenyl)-2-hydroxyethanamin und dessen pharmazeutisch akzeptable Salze.

16. N-[(2R)-7-Methoxycarbonylmethoxy-1,2,3,4-tetrahydronaphth-2-yl]-(2R)-2-(3-chlorphenyl)-2-hydroxyethanamin und dessen pharmazeutisch akzeptable Salze.

**Patentansprüche für folgenden Vertragsstaat : ES**

1. Verfahren zur Herstellung von Verbindungen der Formel

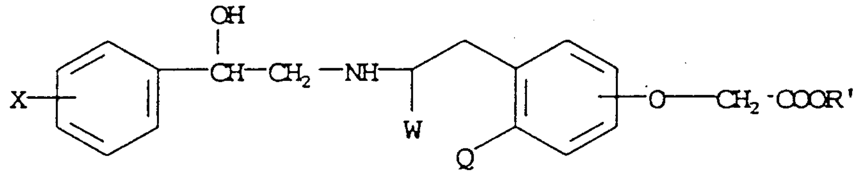

I ,

worin X Wasserstoff, ein Halogen, eine Trifluormethylgruppe oder eine nied.Alkylgruppe darstellt, W eine Methylgruppe darstellt, Q Wasserstoff darstellt, oder W und Q zusammen eine Ethylengruppe bilden und R' eine nied.Alkylgruppe darstellt, sowie deren pharmazeutisch akzeptablen Salzen, dadurch gekennzeichnet, daß

(a) die Aminogruppe einer Verbindung der Formel

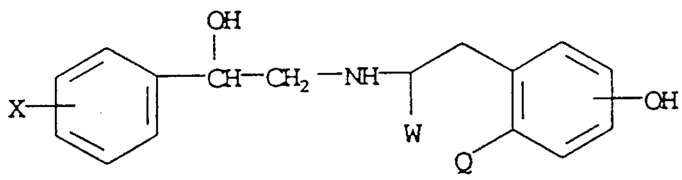

II ,

worin X, W und Q wie oben definiert sind, geschützt wird;

(b) die so erhaltene Verbindung der Formel

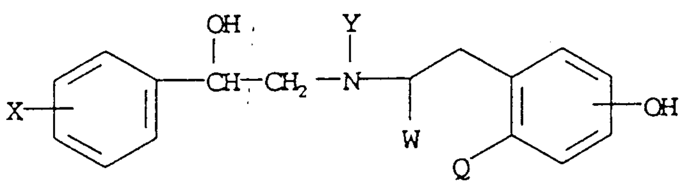

III,

worin X, W und Q wie oben definiert sind und Y eine N-Schutzgruppe darstellt, einer Alkylierung mit einer Verbindung der Formel Hal-$CH_2$-COOR' unterzogen wird, worin R' wie oben definiert ist und Hal für Chlor, Brom oder Iod steht;

c) die geschützte Aminogruppe der so erhaltenen Verbindung der Formel

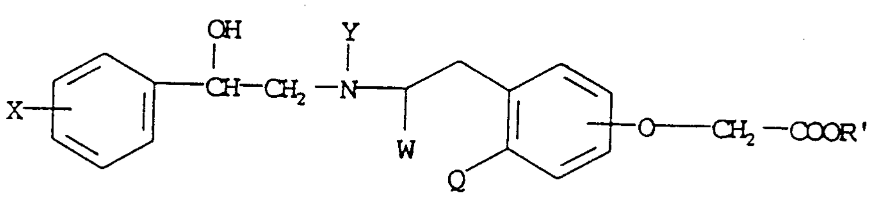

IV,

worin X, Y, W, Q und R' wie oben definiert sind, entschützt wird,

und das so erhaltene Produkt gegebenenfalls in eines seiner pharmazeutisch akzeptablen Salze übergeführt wird.

2. Verfahren nach Anspruch 1, dadurch gekennzeichnet, daß die Schutzgruppe Benzyloxycarbonyl oder tert.Butyloxycarbonyl ist.

3. Verfahren nach einem der Ansprüche 1 oder 2, dadurch gekennzeichnet, daß als Verbindung der Formel Hal-$CH_2$-COOR' Methyl- oder Ethylbromacetat verwendet wird.

4. Verfahren zur Herstellung einer Verbindung der Formel

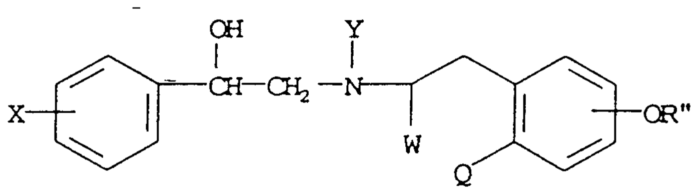

V,

worin X Wasserstoff, ein Halogen, eine Trifluormethylgruppe oder eine nied.Alkylgruppe darstellt, W eine Methylgruppe darstellt, Q Wasserstoff darstellt, oder W und Q zusammen eine Ethylengruppe bilden, R'' Wasserstoff oder eine Gruppe $CH_2$-COOR' darstellt, wobei R' eine nied.Alkylgruppe ist, und Y eine Schutzgruppe darstellt, dadurch gekennzeichnet, daß

(a) die Aminogruppe einer Verbindung der Formel

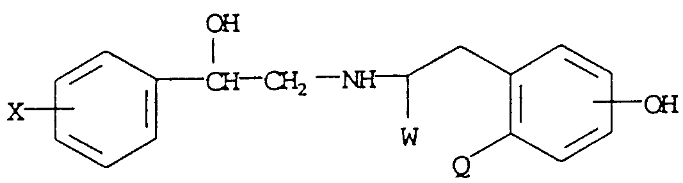

II ,

worin X, W und Q wie oben definiert sind, geschützt wird;

(b) die so erhaltene Verbindung der Formel

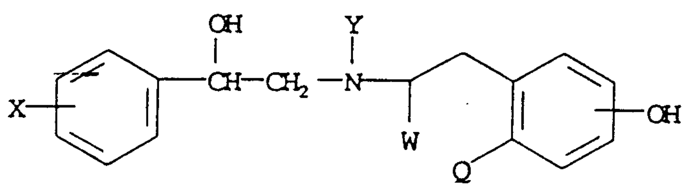

III ,

worin X, W und Q wie oben definiert sind und Y eine N-Schutzgruppe darstellt, gegebenenfalls einer Alkylierung mit einer Verbindung der Formel Hal-$CH_2$-COOR' unterzogen wird, worin R' wie oben definiert ist und Hal für Chlor, Brom oder Iod steht, um die Verbindung der Formel

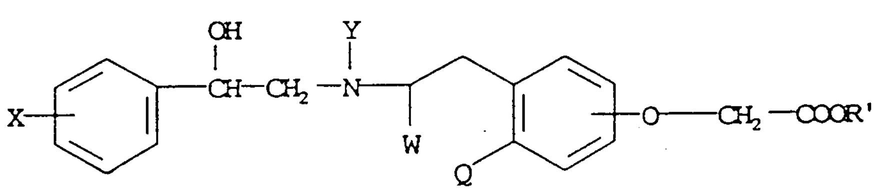

IV

zu bilden.

5. Verfahren nach Anspruch 4, dadurch gekennzeichnet, daß die Aminogruppe durch Umsetzung mit Di-tert.butyldicarbonat in basischem Medium geschützt wird.

6. Verfahren nach einem der Ansprüche 4 oder 5, dadurch gekennzeichnet, daß N-(7-Hydroxy-1,2,3,4-tetrahydronaphth-2-yl)-2-(3-chlorphenyl)-2-hydroxyethanamin als Verbindung der Formel II zur Bildung von N-tert.Butyloxycarbonyl-N-(7-hydroxy-1,2,3,4,-tetrahydronaphth-2-yl)-2-(3-chlorphenyl)-2-hydroxyethanamin verwendet wird.

7. Verfahren nach einem der Ansprüche 4 oder 5, dadurch gekennzeichnet, daß N-(7-Hydroxy-1,2,3,4-tetrahydronaphth-2-yl)-2-(3-chlorphenyl)-2-hydroxyethanamin als Verbindung der Formel II und eine Verbindung der Formel Hal-$CH_2$-COOR', worin R' Ethyl ist, zur Bildung von N-tert.Butyloxycarbonyl-N-(7-ethoxycarbonylmethoxy-1,2,3,4-tetrahydronaphth-2-yl)-2-(3-chlorphenyl)-2-hydroxyethanamin verwendet wird.

8. Verfahren nach einem der Ansprüche 4 oder 5, dadurch gekennzeichnet, daß N-[2-(4-Hydroxyphenyl)-1-methylethyl]-2-(3-chlorphenyl)-2-hydroxyethanamin als Verbindung der Formel II zur Bildung von N-tert.Butyloxycarbonyl-N-[2-(4-hydroxyphenyl)-1-methylethyl]-2-(3-chlorphenyl)-2-hydroxyethanamin verwendet wird.

9. Verfahren nach einem der Ansprüche 4 oder 5, dadurch gekennzeichnet, daß N-[2-(4-Hydroxyphenyl)-1-methylethyl]-2-(3-chlorphenyl)-2-hydroxyethanamin als Verbindung der Formel II und eine Verbindung der Formel Hal-$CH_2$-COOR', worin R' Ethyl ist, zur Bildung von N-tert.Butyloxycarbonyl-N-[2-(4-ethoxycarbonylmethoxyphenyl)-1-methylethyl]-2-(3-chlorphenyl)-2-hydroxyethanamin verwendet wird.

10. Verfahren nach einem der Ansprüche 4 oder 5, dadurch gekennzeichnet, daß N-[2-(4-Hydroxyphenyl)-1-methylethyl]-2-(3-chlorphenyl)-2-hydroxyethanamin als Verbindung der Formel II und eine Verbindung der Formel Hal-$CH_2$-COOR', worin R' Methyl ist, zur Bildung von N-tert.Butyloxycarbonyl-N-[2-(4-methoxycarbonylmethoxyphenyl)-1-methylethyl]-2-(3-chlorphenyl)-2-hydroxyethanamin verwendet wird.

11. Verfahren nach einem der Ansprüche 4 oder 5, dadurch gekennzeichnet, daß N-[(2R)-7-hydroxy-1,2,3,4-tetrahydronaphth2-yl]-(2R)-2-(3-chlorphenyl)-2-hydroxyethanamin als Verbindung der Formel II zur Bildung von N-tert.Butyloxycarbonyl-N-[(2R)-7-hydroxy-1,2,3,4-tetra-hydronaphth-2-yl]-(2R)-2-(3-chlorphenyl)-2-hydroxyethanamin verwendet wird.

12. Verfahren nach einem der Ansprüche 4 oder 5, dadurch gekennzeichnet, daß N-[(2S)-7-hydroxy-1,2,3,4-tetrahydronaphth-2-yl]-(2R)-2-(3-chlorphenyl)-2-hydroxyethanamin als Verbindung der Formel II zur Bildung von N-tert.Butyloxycarbonyl-N-[(2S)-7-hydroxy-1,2,3,4-tetra-hydronaphth-2-yl]-(2R)-2-(3-chlorphenyl)-2-hydroxyethanamin verwendet wird.

13. Verfahren nach einem der Ansprüche 4 oder 5, dadurch gekennzeichnet, daß N-[(2S)-7-hydroxy-1,2,3,4-tetrahydronaphth-2-yl]-(2S)-2-(3-chlorphenyl)-2-hydroxyethanamin als Verbindung der Formel II zur Bildung von N-tert.Butyloxycarbonyl-N-[(2S)-7-hydroxy-1,2,3,4-tetra-hydronaphth-2-yl]-(2S)-2-(3-chlorphenyl)-2-hydroxyethanamin verwendet wird.

14. Verfahren nach Anspruch 1, dadurch gekennzeichnet, daß N-[(2R)-7-hydroxy-1,2,3,4-tetrahydronaphth-2-yl]-(2R)-2-(3-chlorphenyl)-2-hydroxyethanamin als Verbindung der Formel II und eine Verbindung der Formel Hal-$CH_2$-COOR', worin R' Ethyl ist, zur Bildung von N-[(2R)-7-Ethoxycarbonylmethoxy-1,2,3,4-tetrahydronaphth-2-yl]-(2R)-2-(3-chlorphenyl)-2-hydroxyethanamin und dessen pharmazeutisch akzeptablen Salzen verwendet wird.

15. Verfahren nach Anspruch 1, dadurch gekennzeichnet, daß N-[(2S)-7-hydroxy-1,2,3,4-tetrahydronaphth-2-yl]-(2R)-2-(3-chlorphenyl)-2-hydroxyethanamin als Verbindung der Formel II und eine Verbindung der Formel Hal-$CH_2$-COOR', worin R' Ethyl ist, zur Bildung von N-[(2S)-7-Ethoxycarbonylmethoxy-1,2,3,4-tetrahydronaphth-2-yl]-(2R)-2-(3-chlorphenyl)-2-hydroxyethanamin und dessen pharmazeutisch akzeptablen Salzen verwendet wird.

16. Verfahren nach Anspruch 1, dadurch gekennzeichnet, daß N-[(2R)-7-hydroxy-1,2,3,4-tetrahydronaphth-2-yl]-(2R)-2-(3-chlorphenyl)-2-hydroxyethanamin als Verbindung der Formel II und eine Verbindung der Formel Hal-$CH_2$-COOR', worin R' Methyl ist, zur Bildung von N-[(2R)-7-Methoxycarbonylmethoxy-1,2,3,4-tetrahydronaphth-2-yl]-(2R)-2-(3-chlorphenyl)-2-hydroxyethanamin und dessen pharmazeutisch akzeptablen Salzen verwendet wird.